# EUROPEAN PATENT SPECIFICATION

(11) **EP 2 612 647 B1**
(45) Date of publication and mention of the grant of the patent: **13.07.2016**
(21) Application number: 12194061.3
(22) Date of filing: 21.03.2008
(51) Int. Cl.: A61K 8/04, A61K 8/26, A61K 8/81, A61Q 5/02, A61Q 19/00, A61Q 19/10

(54) **Structured compositions comprising a clay**
Strukturierte Zusammensetzungen mit Tonerde
Compositions structurées comprenant de l'argile

(30) Priority: 21.03.2007 US 896146 P
(43) Date of publication of application: 10.07.2013
(62) Divisional of application: 08732652.6
(73) Proprietor: Colgate-Palmolive Company, New York, NY 10022 (US)
(72) Inventor: Hilliard, JP., Peter R., Far Hills, NJ New Jersey 07931 (US); Hassan, Mahmoud, Somerset, NJ New Jersey 08873 (US); Kaplan, Stacey, East Brunswick, NJ New Jersey 08816 (US); Soliman, Nadia, East Brunswick, NJ New Jersey 08816 (US)
(74) Representative: Jenkins, Peter David

(56) References cited:
- US-A1- 2005 227 880

## Description

### BACKGROUND OF THE INVENTION

The present invention relates to structured compositions, and in particular to personal care compositions comprising a clay.

Standard personal care compositions such as cleansing formulas, liquid soaps, moisturizing formulations, hair care products, shower gels and body washes often do not deliver significant amounts of desired products, such as emollients and skin softening agents, to the surface of the skin during use. This can lead to significant drying of the skin, particularly during the winter months and in dry climates. Formulas that deliver higher amounts of oil may temporarily alleviate drying, although they often present the disadvantage of leaving a greasy or oily residue on the skin even after the compositions are washed off. Additionally, formulas with high levels of oils and emollients are often expensive, and can exhibit undesirable separation of the oil and aqueous phases during storage.

Some known personal care compositions such as liquid soaps, shower gels and body washes incorporate a high emollient concentration. This can cause the problem that excessive amounts of oily deposits are formed on sanitary ware, such as shower cubicles, baths and wash basins, requiring regular cleaning of the sanitary ware.

Accordingly, there is an ongoing need for personal care compositions that deliver significant amounts of oils and emollients to the skin during use, and effectively prevent drying of the skin without leaving a greasy or oily residue.

In addition, there is a desire for personal care compositions such as liquid soaps, shower gels and body washes that can deliver a high degree of moisturizing benefit, for example from emollients, to the skin, but which do not tend to deposit excessive amounts of oily deposits onto sanitary ware, such as shower cubicles, baths and wash basins.

US-A-2005/227880 discloses an isotropic cleaning composition with particulate optical modifiers.

### BRIEF SUMMARY OF THE INVENTION

The present invention provides a personal care composition comprising:
a. a structured aqueous gel component formed from 0.1 to 1%, optionally 0.1 to 0.5% of a clay; 1 to 7.5% of a crosslinked polycarboxylate thickener; and 0.1 to 2% of quaternary polymer, each wt% being based on the weight of the composition; and
b. 0.1 to 25%, based on the weight of the composition, of an emollient component, the emollient component being dispersed in the gel component,
wherein the clay comprises a synthetic layered silicate, optionally the personal care composition is a body wash, a shower gel or a liquid hand soap.

Preferred features are defined in the dependent claims.

The clay may comprise a sodium magnesium silicate, in particular a synthetic hectorite, such as a clay available under the trade name Laponite™ available from Southern Clay Products (Gonzales, Texas, USA).

A particularly preferred Laponite™ clay is Laponite™ XLG.

In one embodiment, the crosslinked polycarboxylate thickener comprises an acrylic crosslinked polycarboxylate thickener.

In one embodiment, the quaternary polymer has a molecular weight of less than about 2,000,000 and a charge density of less than about 6 meq/g at a pH of about 7.

In one embodiment, the structured aqueous gel component is comprised in a first visually distinct area and the composition further comprises a second visually distinct area comprising a second structured aqueous gel component formed from 0.1 to 1% optionally 0.1 to 0.5% of a clay; 1 to 7.5% of a crosslinked polycarboxylate thickener, 0.1 to 2%; optionally 1 to about 2% of quaternary polymer, each wt% being based on the weight of the second visually distinct area, wherein the clay comprises a synthetic layered silicate; and wherein the emollient component comprises 0.1 to 20% by weight of the composition, the emollient component being dispersed in at least one of the first and second gel components.

In one embodiment, the emollient component is dispersed in both of the first and second gel components.

The clay may comprise a sodium magnesium silicate, in particular a synthetic hectorite, such as a clay available under the trade name Laponite™. A particularly preferred Laponite™ clay is Laponite™ XLG.

In one embodiment, each of the first and second structured aqueous gel components has the same composition of clay, crosslinked polycarboxylate thickener and quaternary polymer, and the same amount of the emollient component, and the first and second structured aqueous gel components are visually distinct by comprising different colorant compositions therein.

The first and second structured aqueous gel components may comprise different colorant compositions either by the first and second structured aqueous gel components comprising different colorants and/or different concentrations of the same colorants or by the first structured aqueous gel component comprising at least one colorant and the second structured aqueous gel component being about free of any colorant.

In another aspect, the present invention provides a method of cleaning a keratinous surface, said method comprising the steps of applying a composition according to the present invention to the keratinous surface and rinsing the composition from the keratinous surface.

In another aspect, the present invention provides a method of conditioning a keratinous surface, said method comprising the steps of applying a composition according to the present invention to the keratinous surface and rinsing the composition from the keratinous surface.

The keratinous surface may be chosen from skin, hair, or nails.

In another aspect, the present invention provides a method of preventing dry skin or dry scalp, comprising the steps of: applying to the skin or scalp a composition comprising a composition according to the present invention; and rinsing the composition from the skin or scalp.

In another aspect, the present invention provides a method of prolonging delivery of moisture to a keratinous surface, comprising applying to the keratinous surface a composition in accordance with the present invention.

In another aspect, the present invention provides a method of producing personal care composition comprising:
a. dispersing a clay in water to form a composition;
b. adding an anionic surfactant to the composition in which the anionic surfactant is bound to the clay;
c. adding a crosslinked polycarboxylate thickener to the composition to form a structured phase;
d. adding a quaternary polymer to the structured phase to form a structured gel component; and
e. adding at least one emollient to the structured gel component..

In one embodiment, at least one amphoteric surfactant is added to the structured phase formed in step (c) before addition of the quaternary polymer in step (d).

In one embodiment, at least one preservative is added to the structured phase formed in step (c) before addition of the quaternary polymer in step (d), or after the addition of the emollients.

In one embodiment, the at least one preservative is added to the structured phase formed in step (c) before the addition of the at least one amphoteric surfactant.

In one embodiment, at least one pH adjuster is added to the structured phase formed in step (c) before addition of the quaternary polymer in step (d), or before the addition of the at least one amphoteric surfactant.

Also disclosed herein is a personal care composition comprising a single structured gel phase, having at least two visually distinct areas, each visually distinct area comprising a respective structured aqueous gel component comprising a respective visual additive imparting a respective individual visual appearance to the respective structured aqueous gel component, at least one benefit agent selected from a moisturizing agent and a cleaning agent, the at least one benefit agent being dispersed throughout the at least two visually distinct areas whereby the at least two gel components commonly deliver at least one of a cleaning benefit and a moisturizing benefit.

In one embodiment, the composition comprises the moisturizing agent and the cleaning agent, both the moisturizing agent and the cleaning agent being dispersed throughout the at least two visually distinct areas whereby the at least two structured aqueous gel components commonly deliver both the cleaning benefit and the moisturizing benefit.

In one embodiment, the cleaning agent comprises at least one surfactant and the moisturizing agent comprises at least one emollient, and the at least one surfactant and at least one emollient are dispersed throughout the at least two visually distinct areas, and dispersed throughout the composition.

Also disclosed herein is a personal care composition comprising:
a. about 0.01 to about 5% of a clay;
b. about 0.1 to about 10% of a crosslinked polycarboxylate thickener;
c. about 0.01 to about 5% of quaternary polymer;
d. about 0.1 to about 20% of an emollient;
e. about 0.05 to about 10% of a polyhydric alcohol having a molecular weight of less than about 600; and
f. optionally, about 0.05 to about 5% of an antibacterial agent;
wherein the composition has a viscosity at room temperature of about 20,000 to about 100,000 mPas (cps), and a yield point at room temperature of greater than about 5 Pa.

Also disclosed herein is a composition comprising:
a. a synthetic or natural clay;
b. a crosslinked polycarboxylate thickener;
c. a quaternized polymer;
d. a surfactant mixture comprising at least one anionic surfactant and at least one amphoteric surfactant; and
e. a polyhydric alcohol having a molecular weight of less than about 600;
wherein the composition has a viscosity at room temperature of about 10,000 to about 100,000 mPas (cps), and a yield point at room temperature of greater than about 5 Pa.

Also disclosed herein is a personal care composition comprising:
a. a first visually distinct area comprising a synthetic or natural clay; and
b. a second visually distinct area;
wherein at least one of the first or second visually distinct areas comprises a material chosen from a crosslinked polycarboxylate thickener, a quaternized polymer and an antibacterial agent.

Also disclosed herein is a personal care composition comprising:
a. a first visually distinct area comprising a clay, a crosslinked polycarboxylate thickener, a quaternized polymer, optionally an antibacterial agent, and, optionally a first colorant; and
b. a second visually distinct area comprising a clay, a crosslinked polycarboxylate thickener, a quaternized polymer, optionally an antibacterial agent and, optionally a colorant that is the same as or different than the first colorant, and optionally a second colorant.

Also disclosed herein is a method of conditioning a keratinous surface, comprising the steps of:
I) applying to the keratinous surface a composition comprising:
   about 0.01 to about 5% of a synthetic or natural clay;
   a crosslinked polycarboxylate thickener;
   a quaternary polymer;
   a surfactant mixture comprising at least one anionic surfactant and at least one amphoteric surfactant, and
   an emollient;
   wherein the composition has a viscosity at room temperature of about 20,000 to about 100,000 mPas (cps), and a yield point at room temperature of greater than about 5 Pa; and
II) rinsing the composition from the keratinous surface.

In another aspect, a body wash comprising at least one surfactant and at least 1 weight % of an emollient that is solid below 50°C that deposits less than 2 mg/cm² of the emollient on glass according to the Method for Residue Deposition on Glass for Liquid Body Cleansing Products.

### BRIEF DESCRIPTION OF THE DRAWINGS

Figure 1 shows the relationship between the yield point, measured in Pascals and various commercially available PolyQuaternium compounds as used in the Formulae A to I.
Figure 2 shows the relationship between the yield point, measured in Pascals as for the results of Figure 1, and charge density for various commercially available PolyQuaternium compounds as used in the Formulae A to I.
Figure 3 shows the relationship between the yield point, measured in Pascals as for the results of Figure 1, and molecular weight for various commercially available PolyQuaternium compounds as used in the Formulae A to I.
Figure 4 shows the relationship between the yield point, measured in Pascals as for the results of Figure 1, and the amount, in wt% (as supplied) based on the weight of the composition, of a commercially available PolyQuaternium compound, PolyQuat 7.
Figure 5 shows the relationship between order of addition and chemical interactions of the clay, surfactant, and polycarboxylate as measured using Raman spectroscopy for two structured gels formed in accordance with the present invention.
Figure 6 shows the deposition of petrolatum from an inventive composition onto wool based on the weight % of petrolatum in the composition.
Figure 7 shows perceived arm moisturization upon drying after one wash with a shower gel product as a function of weight % petrolatum.
Figure 8 shows the change in viscosity for an inventive composition based on the weight % of cocamidopropyl betaine in the composition.

### DETAILED DESCRIPTION OF THE INVENTION

As used throughout the present disclosure, ranges are a shorthand for describing each and every value that is within the range. Any value within the range can be selected as the terminus of the range. In the event of any conflict between a definition in the present disclosure and that of a cited reference, the present disclosure controls.

As used throughout this specification and claims, except as noted below in the examples, the amount of material listed is the active weight of the material.

As used herein, the term "yield point" (used interchangeably with "yield value") refers to a measurement of structure forming potential of a formula, *i.e.,* the ability to suspend materials (such as oils, beads, etc.) with densities that differ from those of the base material. The compositions of the present invention have yield points of greater than about 5 Pa at room temperature, as measured using a Brookfield YR-1 Yield Rheometer with a number 73 vane. In various embodiments, the yield point of the compositions of the present invention are about 10 to about 100 Pa, about 20 to about 80 Pa, and about 30 to about 70 Pa.

As used throughout, "room temperature" refers to 23°C±1.

As used throughout, the viscosity is measured in mPas (cps) at room temperature with a Brookfield DV-II viscometer using a number 6 spindle rotating at 10 rpm for 30 seconds.

It has been discovered that certain compositions comprising a clay, a crosslinked polycarboxylate thickener and a quaternized polymer are advantageous as personal care formulations. In addition, such compositions may be useful as personal care compositions that exhibit aesthetically desirable attributes, such as, e.g., visually distinct areas.

In particular, the present invention is at least partly predicated on the finding by the present inventors that the provision of a combination of three structurants in an aqueous composition can provide the combination of a highly structured composition with a desirable rheology to provide packaging and aesthetic benefits together with an ability to support benefit agents, in particular emollients to deliver a high level of moisturization for personal care products. The three structurants comprise the clay, particularly a layered silicate clay, the crosslinked polycarboxylate thickener; and the quaternary polymer.

Such a system forms a structured gel phase, but at very low amounts of clay, typically from 0.1 to 1 wt%, more typically 0.1 to 0.5 wt%, yet more typically about 0.3 wt%, clay based on the weight of the composition. This means that the composition may be clear or opaque, depending on the other components in the composition apart from the system forming the structured gel, and can have good aesthetic properties, visual and tactile, for a personal care composition. Also, the low amount of clay reduces the cost of the composition. Yet further, the use of the low clay content structured gel phase significantly reduces the cost of providing a personal care composition that can support a high emollient oil content, to deliver a high degree of moisturization when applied to the skin, as compared to many current commercial personal care bodywash, shower gel or liquid soap compositions. The aqueous composition can have a high water content, typically more than about 50 wt% of the composition.

The clay, particularly the layered silicate clay, can form a colloidal dispersion in water which can provide rheology modification for the aqueous composition so as to provide a thickened product with high shear thinning and a thixotropic rheology. The addition of a crosslinked polycarboxylate thickener, in particular an anionic thickener thereto can modify the rheology to stabilize and thicken the composition. The further addition of the third component, the quaternary polymer, greatly increases the yield point of the composition so that the formulation has a very high shear and is mechanically stable after manufacture and during transport to the consumer, and during use. However, the thixotropic properties of the composition ensure that the composition can readily be manufactured, for example by injection of the liquid composition from a nozzle into a container for delivery to the consumer, and can readily be used by the consumer when dispensing a desired amount or dose of the composition from the container. The quaternary polymer is typically present in an amount of about 0.01 to about 5 wt%, or about 0.05 to about 2 wt%, more typically 0.1 to 0.2 wt%, about 0.108 wt% based on the weight of the composition, to achieve the desired increase in yield point of the structured gel formed from the clay, crosslinked polycarboxylate thickener and quaternary polymer.

Furthermore, benefit agents, in particular emollients to deliver a high level of moisturization for personal care products, are supported by the structured gel phase in a uniformly and highly dispersed manner, and there is no oil-in water emulsion.

Consequently, the personal care compositions can support a high proportion of emollients, typically about 3 to about 10 wt%, more typically about 5 wt%, emollient based on the weight of the composition, which can correspondingly deliver a high degree of moisturization to the skin when the composition is used, for example, as a body wash, shower gel or liquid hand soap, without the composition feeling oily or greasy to the touch.

Furthermore, even with a high amount of emollient the composition visually appears to have a consistent and uniform single phase composition, even if different visually distinct areas, distinguished by different coloration, for example, are provided. This is achievable in highly aqueous compositions, for example where water as a solvent for the gel phase comprises at least about 50 wt% of the composition. High water content reduces the manufacturing cost of the composition.

The visually distinct areas may for example be achieved by simultaneously co-injecting the two visually distinct structured gel components into a common container or package from respective nozzles or from a single nozzle.

In addition, the provision of the highly structured gel phase provides a base formulation that can be employed in a number of different personal care compositions, thereby reducing product development periods, and formulation and manufacturing costs, across a range of different products. For example, the personal care composition can comprise liquid hand soaps, shower gels or bodywashes using the same base formulation, the products primarily varying in color and/or fragrance, and possibly also emollient content because the range of emollient content that can be supported within the composition is rather large, for example up to about 15 wt% based on the weight of the composition.

The highly structured gel permits plural visually distinct areas to be present in the same container or package, and the areas can remain visually distinct during transport of the product from the manufacturer to the consumer because of the high yield point of the gel. There is no need to formulate the structured gels differently to provide two visually distinct areas, apart from providing different visual differences, such as differential coloration. This simplifies manufacture and reduces the manufacturing cost. However, the thixotropic nature of the gel readily permits both filling of the container or package during manufacture of the product and subsequent consumer acceptable dispensing of the composition from the container or package during use by the consumer.

In addition, it has been found that the structured gel can support a high amount of emollient which can deliver a high degree of moisturization to the skin yet without causing emollient, in the form of oil, to be deposited in significant amounts onto sanitary ware, such as bathtubs, shower cubicles and wash basins, which is clearly undesirable.

Some known personal care compositions, such as bodywashes and shower gels, that contain emollients for skin moisturizing can deliver a high degree of moisturization to the skin but correspondingly they also cause significant amounts of emollient to be deposited onto sanitary ware, which then requires frequent cleaning.

The present inventors have found that the personal care compositions of the present invention, incorporating the structured gel, support emollient at highly moisturizing levels so as to be able to provide, when used as a bodywash, shower gel or soap, a high deposition of emollient on the skin with reduced deposition on sanitary ware such as bathtubs, shower cubicles and wash basins, which may be made of ceramic, glass, or plastics material, such as acrylic plastics. Such differential deposition of emollient onto skin as compared to onto sanitary ware is a significant technical advantage of the compositions of the preferred embodiments of the present invention.

One particular advantage of the personal care compositions of the present invention is that two or more visually distinct areas can be provided which creates an attractive aesthetic appearance to the consumer. Moreover, the aesthetic appearance of two or more visually distinct areas can impart to the consumer the technical concept of the composition providing plural technical effects, for example moisturization and a cleaning action, each associated with a respective visually distinct area. Such a technical concept can be imparted even though the visually distinct areas have essentially the same composition, apart from those components that provide the visual distinction, such as different colorants or colorant contents.

This in turn can provide the advantage that when, for example, an emollient system is incorporated into the composition, the emollient can be equally present in each visually distinct area, and so can be uniformly distributed throughout the composition, even though the consumer may visually perceive there to be only one of the visually distinct areas that would be expected to be formulated to provide the moisturizing effect. This means that a high level of emollient can be incorporated into the composition having two or more visually distinct areas without having to provide a relatively high emollient concentration phase and a relatively low, or even zero, emollient concentration phase, as in some known multiple phase compositions, and deliver better perceived benefits, aesthetics, and rheology.

Such known compositions may exhibit excessive emollient deposition onto sanitary ware, as discussed above, because of the need for an excessively high emollient content in one phase. Such known compositions may comprise an aqueous phase that comprises surfactants and delivers a cleaning benefit and an anhydrous phase or emulsion phase (a water-in-oil or oil-in-water emulsion) that comprises emollients and delivers a moisturizing benefit.

In the present invention, there can be a single structured gel phase, having two visually distinct areas, commonly delivering both a cleaning benefit and a moisturizing benefit because both surfactants and emollients are dispersed throughout both visually distinct areas.

As used herein, the term "structured" refers to a composition in which the base, active material and structuring agent form a system with solid suspending properties while remaining pourable. Examples of structured systems include those wherein the active materials (such as detergents, surfactants, emollients, moisturizers and the like) are dispersions of lamellar droplets in an aqueous phase that contains an electrolyte. These lamellar droplets are often referred to as an "onion-like" configuration or layering of surfactant molecules, for example, as spherulites. *See, e.g.,* U.S. Patent Publication Nos. 2004/0092415, 2004/0223991, 2004/0235693 and 2004/0248748 which are directed to spherulite-based structured systems.

The structured compositions of the present invention contain a clay, and are hence directed to clay-based (rather than spherulite-based) structurant systems. As used herein, the term "clay" refers to any of a series of hydrous silicate minerals and includes natural or synthetic clays. Examples of useful classes of clays include, but are not limited to: kaolinites, smectites, illites and chlorites. In certain embodiments of the present invention, the clay may be useful as a thickener and/or structure building composition. For example, swelling clays such as smectites are particularly useful as structurants, and include, e.g., bentonite, hectorite, layered magnesium silicate (such as a clay available from Southern Clay Products (Gonzales, Texas, USA) under the trade name Laponite™); and magnesium aluminum silicate (such as a clay available under the trade name Veegum from various suppliers, USA). U.S. Patent No. 6,787,160 to Schacknai et al. provides further discussion of natural and synthetic clays. A synthetic layered silicate, such as Laponite™ XLG, which is anionic, is preferred. In various embodiments of the present invention, the amounts of clay present are about 0.01 to about 5%, about 0.05 to about 3%, about 0.1 to about 2% and about 0.2 to about 1% by weight of the total composition.

The compositions of the present invention may comprise a mixture of surfactants, comprising at least one anionic surfactant and at least one amphoteric surfactant. Suitable surfactants are described in McCutcheon's, Detergents and Emulsifiers, North American edition (1986), published by allured Publishing Corporation; and McCutcheon's, Functional Materials, North American Edition (1992); and in U.S. Pat. No. 3,929,678.

Useful anionic surfactants for the present embodiments include alkyl and alkyl ether sulfates, such as those that may have the respective formula ROSO₃M and RO(C₂H₄O)_{X}SO₃M, wherein R is alkyl or alkenyl of from about 8 to about 24 carbon atoms, x is 1 to 10, and M is a water-soluble cation such as ammonium, sodium, potassium and triethanolamine. The alkyl ether sulfates may be made as condensation products of ethylene oxide and monohydric alcohols having from about 8 to about 24 carbon atoms. In one embodiment, R has from about 10 to about 18 carbon atoms in both the alkyl and alkyl ether sulfates. The alcohols can be derived from fats, e.g., coconut oil or tallow, or can be synthetic. Lauryl alcohol and straight chain alcohols derived from coconut oil are preferred herein. Such alcohols are reacted with about 1 to about 10, or about 3 to about 5, or with about 3, molar proportions of ethylene oxide and the resulting mixture of molecular species having, for example, an average of 3 moles of ethylene oxide per mole of alcohol, is sulfated and neutralized.

Specific examples of alkyl ether sulfates include sodium and ammonium salts of coconut alkyl triethylene glycol ether sulfate; tallow alkyl triethylene glycol ether sulfate, and tallow alkyl hexaoxyethylene sulfate. Highly preferred alkyl ether sulfates are those comprising a mixture of individual compounds, said mixture having an average alkyl chain length of from about 10 to about 16 carbon atoms and an average degree of ethoxylation of from about 1 to about 4 moles of ethylene oxide.

Other suitable anionic surfactants include water-soluble salts of the organic, sulfuric acid reaction products of the general formula [R₁-SO₃-M], wherein R₁ is chosen from a straight or branched chain, saturated aliphatic hydrocarbon radical having from about 8 to about 24, or about 10 to about 18, carbon atoms; and M is a cation. Suitable examples include the salts of an organic sulfuric acid reaction product of a hydrocarbon of the methane series, including iso-, neo-, ineso-, and n-paraffins, having about 8 to about 24 carbon atoms, or about 10 to about 18 carbon atoms and a sulfonating agent, e.g., SO₃, H₂SO₄, oleum, obtained according to known sulfonation methods, including bleaching and hydrolysis, for example, alkali metal and ammonium sulfonated C₁₀₋₁₈ n-paraffins.

Useful anionic surfactants include ammonium lauryl sulfate, ammonium laureth sulfate, triethylamine lauryl sulfate, triethylamine laureth sulfate, triethanolamine lauryl sulfate, triethanolamine laureth sulfate, monoethanolamine lauryl sulfate, monoethanolamine laureth sulfate, diethanolamine lauryl sulfate, diethanolamine laureth sulfate, lauric monoglyceride sodium sulfate, sodium lauryl sulfate, sodium laureth sulfate, potassium laureth sulfate, sodium lauryl sarcosinate, sodium lauroyl sarcosinate, lauryl sarcosine, cocoyl sarcosine, ammonium cocoyl sulfate, ammonium lauroyl sulfate, sodium cocoyl sulfate, sodium lauroyl sulfate, potassium cocoyl sulfate, potassium lauryl sulfate, monoethanolamine cocoyl sulfate, sodium tridecyl benzene sulfonate, sodium dodecyl benzene sulfonate, sodium and potassium salts of sodium pareth sulfate, sodium and potassium salts of sodium pareth ether sulfate and combinations thereof.

A particularly preferred anionic surfactant is an ethoxylated sodium pareth sulfate, in particular SLES(SO3Na Pareth 145-2EO Sulfate Base-25.5% AI). Another preferred anionic surfactant is an ethoxylated sodium laureth sulfate, in particular SLES( SO3Na Laureth c12-14 Alcohol-2EO Sulfate Base-70% or 25.5% AI)

Useful amphoteric surfactants include those that may be described as derivatives of aliphatic secondary and tertiary amines in which the aliphatic radical can be straight or branched chain and wherein one of the aliphatic substituents contains from about 8 to about 18 carbon atoms and one contains an anionic water solubilizing group, e.g., carboxy, sulfonate, sulfate, phosphate, or phosphonate. Examples of compounds falling within this definition are sodium 3-dodecyl-aminopropionate, sodium 3-dodecylarninopropane sulfonate, sodium lauryl sarcosinate, N-alkyltaurines such as the one prepared by reacting dodecylamine with sodium isethionate according to the teaching of U.S. Pat. No. 2,658,072, N-higher alkyl aspartic acids such as those produced according to the teaching of U.S. Pat. No. 2,438,091, and the products described in U.S. Pat. No. 2,528,378. Examples of useful amphoteric surfactants include amidobetaines, amidosulfobetaines, coco dimethyl carboxymethyl betaine, cocoamidopropyl betaine, cocobetaine, lauryl amidopropyl betaine, oleyl betaine, lauryl dimethyl carboxymethyl betaine, lauryl dimethyl alphacarboxyethyl betaine, cetyl dimethyl carboxymethyl betaine, lauryl bis-(2-hydroxyethyl) carboxymethyl betaine, stearyl bis-(2-hydroxypropyl) carboxymethyl betaine, oleyl dimethyl gamma-carboxypropyl betaine, and lauryl bis-(2-hydroxypropyl)alpha-carbox- yethyl betaine, coco dimethyl sulfopropyl betaine, stearyl dimethyl sulfopropyl betaine, lauryl dimethyl sulfoethyl betaine, lauryl bis-(2-hydroxyethyl) sulfopropyl betaine.

The compounds of the present invention further comprise a crosslinked polycarboxylate thickener. In one embodiment, the crosslinked polycarboxylate thickener is an acrylic crosslinked polycarboxylate rheology modifier, or an acrylates copolymer or derivative thereof or an acrylates/methacrylate cross polymer, for example an acrylates steareth-20 methacrylate crosspolymer. Useful crosslinked polycarboxylate thickeners include, for example, partially crosslinked polycarboxylate thickeners that may be partially substituted with at least one alkyl group, *e.g.,* thickeners, which are anionic or nonionic, commercially available under the trade name Aculyn™ from Rohm & Haas (for example, Aculyn™ 22, Aculyn™ 28, Aculyn™ 33, Aculyn™ 38, Aculyn™ 44, Aculyn™ 46, Aculyn™ 60, Aculyn™ 88 and the like). An anionic crosslinked polycarboxylate thickener, such as Aculyn™ 88, is preferred.

The compounds of the present invention may further comprise a polyhydric alcohol having an average molecular weight of less than about 600. In various embodiments, the average molecular weight may be less than about 550 or less than about 500. Any polyhydric alcohol can be used, but examples of suitable polyhydric alcohols include glycerin (glycerol), ethylene glycol, diethylene glycol, propylene glycol, polypropylene glycol, polyethylene glycol, di- and tri-glycerin and/or poly-glycerin and combinations thereof. It has been found that where the polyhydric alcohol has a molecular weight of less than about 600, the compositions are particularly advantageous in terms of desirability and ease of use for consumers. In addition, the addition of the polyhydric alcohol aids in the dispersion of the compositions during formulation, thereby leading to more efficient processing and higher yield of formula.

The compositions of the present invention may further comprise an optional preservative, such as, for example, EDTA. It has been discovered, that where the amounts of EDTA are varied, *e.g.,* from 0 to about 1%, various characteristics can be optimized as well (for example, foam boost or increased oil deposition).

The compositions of the present invention comprise a quaternized polymer, *i.e.,* a highly charged cationic polymer that may effectively build up the structure of the formula, increase yield point and further enhance the ability of the composition to support oils, emollients, particles and other inclusions in the compositions in a stable formulation. Examples of quaternized polymer that may be useful for the present embodiments include, *e.g.,* the homopolymer of dimethyl diallyl ammonium chloride solid under the trade name MERQUAT™ 100 having a molecular weight lower than 100,000 and the copolymer of dimethyl diallyl ammonium chloride and acrylamide, having a molecular weight greater than 500,000 and sold under the name Merquat™ 500, as well as related compositions available under the following trade names: Merquat™ 5, Merquat™ 280, Merquat™ 550 (Polyquat 7), Merquat™ 2001, Merquat™ 3330 all available from Nalco Company (Napierville, Illinois, USA); Conditioneze NT-2 (available from ISP Corp.), Jaguar (available from Rhodia Corporation).

A particularly preferred quaternary polymer has a charge density at pH 7 of about 1 to about 5 meq/gm, or from about 2 to about 4 meq/gm, or about 3 meq/gm, and a molecular weight of from about 1,000,000 to about 2,000,000, or about 1,600,000. Such a quaternary polymer is provided by Merquat™ 550 (Polyquat 7).

The compositions of the present invention comprise at least one emollient oil that enhances the moisturization of the skin. Examples of such oils include vegetable triglycerides such as maleated castor oils (such as that available under the trade name Ceraphyl™, *e.g.*, Ceraphyl™ RMT, from ISP Corp.), maleated soybean oils, sunflower oils, mineral oils, petrolatum, silicones or silicone elastomers, or mixtures or derivatives thereof.

It has particularly been found by the present inventors that when the personal care compositions of the present invention comprise at least two emollient oils, comprising a mixture of a maleated oil, in particular a maleated castor oil (such as that available under the trade name Ceraphyl™, e.g., Ceraphyl™ RMT, from ISP Corp.), and another emollient selected from at least one of petrolatum, a vegetable oil such as sunflower oil, or a mixture thereof, then the efficacy of emollient moisturization of the skin, particularly after the skin has been dried of aqueous moisture, is increased as compared to when the same other emollient is used without the maleated oil. See example below.

It has further been found by the present inventors that the structured gel comprising the clay, the crosslinked polycarboxylate thickener, and the quaternary polymer sometimes has a high viscosity, particularly after a fragrance has been added thereto, which can present problems for manufacturing, consumer use and consumer acceptance. This can be a problem for personal care compositions such as bodywashes, shower gels and liquid hand soap cleansing products. It has further been found by the present inventors that the addition of fatty esters to the composition can reduce the viscosity of the composition.

In particular, the fatty esters may be selected from at least one of isopropyl myristate, isopropyl palmitate, and isopropyl isostearate.

During manufacture of the composition, it has been found that the order of addition of the components can significantly modify the effectiveness of the fatty ester as a viscosity modifier for the composition. For example, if the fatty ester is added immediately after the addition of the clay to the composition, then this can result in a larger reduction in viscosity of the composition than if the fatty ester is added immediately at the end of the formulation process and after the addition of the fragrance to the composition,

In certain embodiments, the compositions of the present invention may be presented in visually distinct areas, e.g. as clouds, stripes or areas of varying opacity, such as, for example, wherein certain areas contain inclusions. It is important to note that the compositions of the present invention are uniform in composition; however, they may exhibit a visually distinct appearance based on different amounts and/or types of colorants used or inclusions, for example. As used herein, the term "visually distinct" refers to a distinction that is visible to the naked eye at a distance at which a consumer using a personal care formulation would likely view a product. This distance may include, for example, arm's length, a distance from a consumer's eye to a store shelf, or about 10 cm to about 3 m or more. "Visually distinct" may include, for example, areas that are different colors, different shades of a color (*i.e.,* different gradations of a color over the dimensions of the container or package), different opacities, contain different inclusions or particles, or different phases such as solid, liquid or gaseous (*e.g*., air bubbles). Also contemplated within the embodiments of the present invention are compositions wherein one or more of such areas is visually clear and/or contains no colorant. The compositions of the present invention are able to maintain their visually distinct characteristics for prolonged periods of time including storage and transportation, without significant changes in their visual appearance, such as, for example, mixing to the extent that the visual patterns are completely obliterated. In various embodiments, shaking or agitation of the compositions may result in changes to the pattern of visual distinction, but visually distinct areas will still be discernible.

Examples of particles that may be suitable for the present compositions include any discrete and visually distinct forms of matter that may be useful in a personal care composition. For example, useful particles include, without limitation: beads, encapsulates, particles made of polymer materials (*e.g*., plastic, in any desirable shape that appeals to consumers), metals *(e.g.,* foil material or flakes, glitter), minerals *(e.g.,* salts, rocks, pebbles, lava, glass/silica particles, talc), plant materials *(e.g.,* pits or seeds of vegetables or fruits, plant fibers, stalks, stems, leaves or roots) and the like.

The embodiments of the present invention may additionally comprise additional materials such as solubilizers, pH adjusters *(e.g.,* citric acid, HCl, NaOH, KOH), viscosity modifiers *(e.g.,* isopropyl palmitate), salts or other electrolytes *(e.g.,* sodium chloride and other mono-, di- and trivalent salts), preservatives.

The compositions of the present invention may be in the form of any acceptable personal care compositions, including but not limited to: hair care products (*e.g*., shampoos, conditioners, mousses, sprays and hair gels), films, liquid soaps such as hand soaps and santizers, antiperspirants, deodorants, body washes, body gels, creams, lotions, bubble baths, bath powders, bath oils, and other portable forms.

The present invention also provides, in certain embodiments, methods for providing to the skin a moisturizing effect comprising applying to the skin a composition comprising any of the foregoing composition embodiments.

In certain embodiments, the invention is directed to compositions of the present invention incorporated into one or more acceptable carriers. Acceptable carriers for the embodiments of the present invention may be in liquid, semi-solid, solid or gaseous phase, and may vary depending upon the composition and intended uses of a particular compound. Acceptable carriers for the personal care embodiments of the present invention should be ones that are dermatologically acceptable and not harsh when applied to the human skin, e.g., the skin of the scalp or other external regions of the human body for which personal care compositions are generally intended.

Selection of specific carrier components is dependent on the desired product form. It should be understood that any suitable carrier known in the art or to be developed can be provided to the composition, and that the carrier or carriers useful for various embodiments of the present invention will depend upon the specific intended use of the compositions, and that one or more carriers may be suitable for overlapping intended uses.

The compositions of the present invention may also include one or more fragrances. Acceptable fragrances for the present invention include any fragrances that are pleasant and desirable for consumers and do not irritate or otherwise adversely affect the human body.

The compositions of the present invention may additionally include ingredients that may further enhance their desirability for consumers. For example, colorants, pH adjusters, preservatives, pearlescent or opacifying agents, thickening agents, conditioners, humectants, chelating agents/sequestrants, absorbents, abrasives, anticaking agents, anti-aging agents, astringents, antifoaming agents, binders, biological additives, buffering agents, bulking agents, chemical additives, colorants, cosmetic astringents, antimicrobial agents, denaturants, emollients, vitamins, foam boosters, sugars and starches, sugar and starch derivatives, hydrotropes, neutralizing agents, opacifying agents and pigments, plasticizers, propellants, reducing agents, skin tanning agents, skin bleaching agents, skin protectants, sunscreens, sunblocks and similar additives may be included in the compositions described herein and are contemplated by the present invention.

In other embodiments, the present invention provides for a method of providing moisture to the skin comprising the steps of applying a composition to the skin comprising a clay, and rinsing the composition from the skin. The step of "applying" includes actions normally associated with oral care and personal care compositions, and includes, e.g., manually rubbing, massaging, rubbing with an implement such as a sponge or scrubber, towel, pad, cotton ball or the like. Conversely, the step of "removing" may refer to, e.g., rinsing, wiping, rubbing, blow-drying or air-drying.

In another embodiment, the present invention provides a body wash comprising at least one surfactant and at least 1 weight % emollient that is solid below 50°C that deposits less than 2 mg/cm² of the emollient on glass according to the Method for Residue Deposition on Glass for Liquid Body Cleansing Products, which is described below. In other embodiments, the amount of emollient is at least 2, 3, 4, 5, 6, 7, 8, 9, 10, 11, 12, 13, 14, 15, 20, 25, 30, 35, 40, 45, or 50 weight % of the composition.

By way of example, and not limitation, specific embodiments of the present invention are illustrated in the following Examples. In the examples, the amounts of the materials listed are by weight as supplied. In the remainder of the specification and the claims, the amount of material is based on the active weight of the material.

### EXAMPLE I

The following materials shown in Table 1 are mixed together to form compositions in accordance with the present invention and include optional materials. All percentages are by weight. The amount of water is listed, but the amount of water could alternatively be q.s. (quantum suficit).

The following procedure was used for Formulas A-C:
- 1.: Heat water to 40°C.
- 2.: Add clay and stir until fully dispersed, when solution becomes transparent.
- 3.: Heat Solution to 60-65°C.
- 4.: Add crosslinked polycarboxylate associative thickener with constant stirring.
- 5.: Turn off heater.
- 6.: Add SLES (sodium lauryl ether sulfate) with stirring.
- 7.: Add cocoamidopropylbetaine with stirring.
- 8.: Add EDTA with stirring.
- 9.: Reduce solution temperature to below 40°C.
- 10.: Add DMDM Hydantoin with stirring.
- 11.: Adjust solution pH to 8.0 with NaOH and citric Acid.
- 12.: Add Polyquat-7 with stirring.
- 13: Add Sunflower Oil to formula with stirring.
- 14.: Make pre-mix of Petrolatum, Ceraphyl™-RMT, and Silicone Elastomer by melting at about 49°C, and add to solution with stirring.
- 15.: Add fragrance with stirring.
- 16.: Make final adjustment to formula pH with NaOH and/or Citric Acid.

**Table 1**

| Ingredients | A | B | C |
|---|---|---|---|
| Deionized Water | (about 52.374) | (about 52.324) | (about 51.924) |
| Clay | 0.300 | 0.300 | 0.300 |
| Crosslinked Polycarboxylate Associative Thickener | 4.250 | 4.250 | 4.250 |
| SLES(SO3Na Pareth 145-2EO Sulfate Base-25.5% AI) | 26.002 | 26.002 | 26.002 |
| Cocoamidopropyl Betaine (Coco Fatty Acid, Topped, Hydrogenated) | 8.878 | 8.878 | 8.878 |
| PolyQuat-7 | 1.200 | 1.200 | 1.200 |
| Tetrasodium EDTA-39% AI | 0.200 | 0.200 | 0.200 |
| DMDM Hydantoin | 0.500 | 0.500 | 0.500 |
| Ceraphyl™-RMT | 0.050 | 0.100 | 0.500 |
| Sunflower Oil | 3.000 | 3.000 | 3.000 |
| Petrolatum | 1.420 | 1.420 | 1.420 |
| Silicone Elastomer | 0.08 | 0.08 | 0.08 |
| NaOH (50% in H₂O) | 0.600 | 0.600 | 0.600 |
| Citric Acid | 0.146 | 0.146 | 0.146 |
| Fragrance | 1.000 | 1.000 | 1.000 |

### EXAMPLE II

Compositions according to the present invention were formed as follows, using the following procedure for Formulas D and E shown in Table 2:
- 1.: Heat water to 40°C.
- 2.: Add Laponite™-XLG and stir until fully dispersed, when solution becomes transparent.
- 3.: Heat formula to 60-65°C.
- 4.: Add Aculyn™ 88 with constant stirring.
- 5.: Turn off heater.
- 6.: Add SLES with stirring.
- 7.: Add CapBetaine with stirring
- 8.: Add EDTA with stirring.
- 9.: Reduce formula temperature to below 40°C.
- 10.: Add DMDM Hydantoin with stirring.
- 11.: Adjust solution pH to 8.0 with NaOH and citric Acid.
- 12.: Add Polyquat-7 with stirring.
- 13.: Add Sunflower Oil to formula with stirring.
- 14.: Make pre-mix of Petrolatum and Ceraphyl™-RMT by melting at about 49°C, and add to formula with stirring,
- 15.: Add fragrance with stirring.
- 16.: Make final adjustment to formula pH with NaOH and/or Citric Acid.

The following procedure was used to make Formulas F and G shown in Table 2:
- 1.: Heat water to 40°C.
- 2.: Add clay and stir until fully dispersed, when solution becomes transparent.
- 3.: Heat formula to 60-65°C.
- 4.: Add crosslinked polycarboxylate associative thickener with constant stirring.
- 5.: Turn off heater.
- 6.: Add SLES with stirring.
- 7.: Add cocoamidopropylbetaine with stirring
- 8.: Add EDTA with stirring.
- 9.: Reduce formula temperature to below 40°C.
- 10.: Add DMDM Hydantoin with stirring.
- 11.: Adjust solution pH to 8.0 with NaOH and citric Acid.
- 12.: Add Polyquat-7 with stirring.
- 13.: Make pre-mix of Sunflower Oil and Ceraphyl™-RMT and add to formula with stirring.
- 14.: Melt Petrolatum at about 49°C, and add to formula with stirring,
- 15.: Add fragrance with stirring.
- 16.: Make final adjustment to formula pH with NaOH and/or Citric Acid.

**Table 2**

| Ingredients | D | E | F | G |
|---|---|---|---|---|
| Deionized Water | 52.454 | 52.434 | 52.454 | 52.434 |
| Clay | 0.300 | 0.300 | 0.300 | 0.300 |
| Crosslinked Polycarboxylate Associative Thickener | 4.250 | 4.250 | 4.250 | 4.250 |
| SLES(SO3Na Pareth 145-2EO Sulfate Base-25.5% AI) | 26.002 | 26.002 | 26.002 | 26.002 |
| CapBetaine (Cocoamidopropyl Betaine (Coco Fatty Acid, Topped, Hydrogenated)) | 8.878 | 8.878 | 8.878 | 8.878 |
| PolyQuat-7 | 1.200 | 1.200 | 1.200 | 1.200 |
| Tetrasodium EDTA-39% AI | 0.200 | 0.200 | 0.200 | 0.200 |
| DMDM Hydantoin | 0.500 | 0.500 | 0.500 | 0.500 |
| Ceraphyl™-RMT | 0.050 | 0.070 | 0.050 | 0.070 |
| Sunflower Oil | 3.000 | 3.000 | 3.000 | 3.000 |
| Petrolatum | 1.420 | 1.420 | 1.420 | 1.420 |
| NaOH (50% in H₂O) | 0.600 | 0.600 | 0.600 | 0.600 |
| Citric Acid | 0.146 | 0.146 | 0.146 | 0.146 |
| Fragrance | 1.000 | 1.000 | 1.000 | 1.000 |

### EXAMPLE III

Additional compositions were formed, as shown in Tables 3 (clay body wash base) and 4, and their characteristics tested as follows:

**Table 3**

| Ingredients | (wt%) |
|---|---|
| Deionized Water | 50.562 |
| Clay | 0.255 |
| Crosslinked Polycarboxylate Associative Thickener | 4.796 |
| SO3Na Pareth 145-2EO Sulfate Base-25.5% AI | 32.653 |
| Cocoamidopropyl Betaine (Coco Fatty Acid, Topped, Hydrogenated)/Glydant™ Preserved | 10.204 |
| Veresen™, Tetrasodium EDTA-39% AI | 0.204 |
| DMDM Hydantoin | 0.306 |
| Fragrance | 1.020 |
| **Total** | 100.000 |

**Table 4**

| Clay Body Wash Base Structured with various PolyQuats at 0.113% on a solids basis. | | | | |
|---|---|---|---|---|
| Ingredients | Wt% | | Ingredients | Wt% |
| **Formula A** | | | **Formula F** | |
| Clay Body Wash Base | 92.000 | | Clay Body Wash Base | 92.000 |
| Deionized Water | 7.887 | | Deionized Water | 7.515 |
| Merquat™ 5 | 0.113 | | Merquat™ 2001 | 0.485 |
| **Totals** | 100.000 | | **Totals** | 100.000 |
| | | | | |
| **Formula B** | | | **Formula G** | |
| Clay Body Wash Base | 92.000 | | Clay Body Wash Base | 92.000 |
| Deionized Water | 7.754 | | Deionized Water | 6.970 |
| Merquat™ 100 | 0.246 | | Merquat™ 3330 | 1.030 |
| **Totals** | 100.000 | | **Totals** | 100.000 |
| **Formula C** | | | | |
| Clay Body Wash Base | 92.000 | | **Formula H** | |
| Deionized Water | 7.751 | | Clay Body Wash Base | 92.000 |
| Merquat™ 280 | 0.249 | | Deionized Water | 7.490 |
| **Totals** | 100.000 | | Conditioneze™ NT-20 | 0.510 |
| **Formula D** | | | **Totals** | 100.000 |
| Clay Body Wash Base | 92.000 | | | |
| Deionized Water | 7.728 | | **Formula** I | |
| Merquat™ 295 | 0.272 | | Clay Body Wash Base | 92.000 |
| **Totals** | 100.000 | | Deionized Water | 7.887 |
| **Formula E** | | | Jaguar C-17 | 0.113 |
| Clay Body Wash Base | 92.000 | | **Totals** | 100.000 |
| Deionized Water | 6.800 | | | |
| Merquat™ 550 (PolyQuat 7) | 1.200 | | | |
| **Totals** | 100.000 | | | |

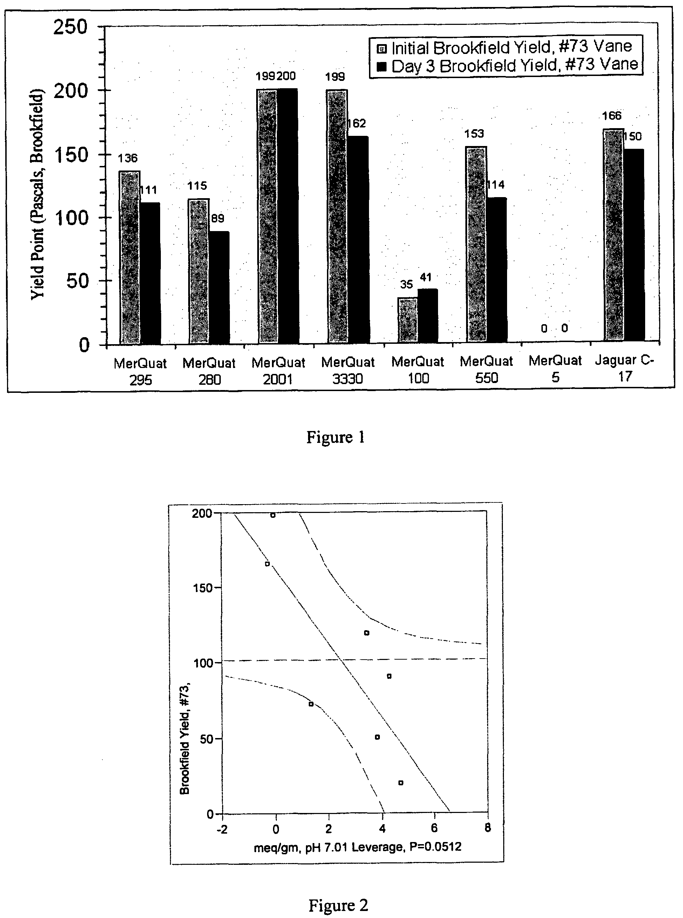

Figure 1 shows the relationship between the yield point, measured in Pascals and various commercially available PolyQuaternium compounds as used in the Formulae A to I above. The yield point was measured using a Brookfield YR-1 Rheometer with a no. 73 vane at room temperature. It may be seen that for the majority of the compositions the yield point was greater than 50 Pascals and for the most preferred compositions the yield point was greater than 100 Pascals. For the majority of compositions the yield point decreased over time, at least over an initial three day period, but the yield point value generally remained at a high value, typically above 100 Pascals.

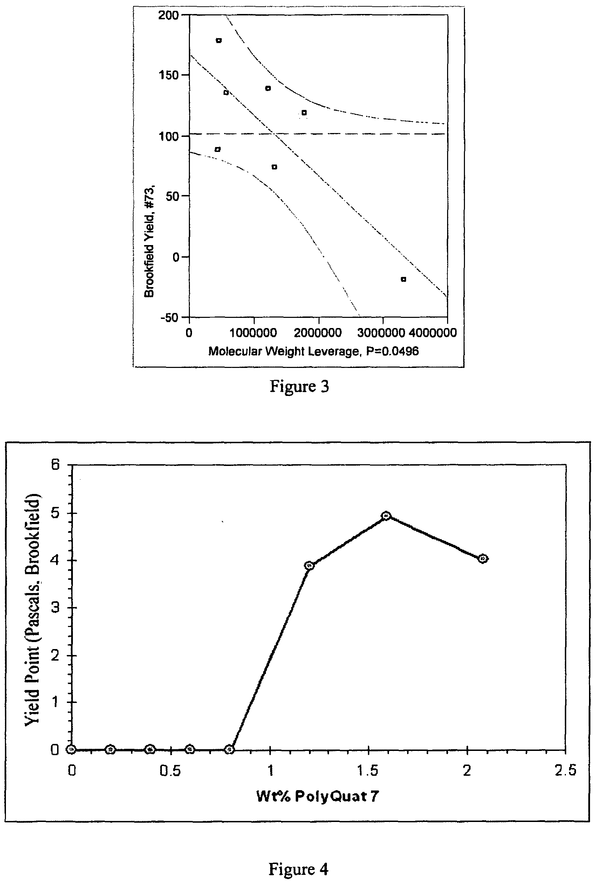

Figures 2 and 3 show the relationship between the yield point, measured in Pascals as for the results of Figure 1, and, respectively, charge density and molecular weight for various commercially available PolyQuaternium compounds as used in the Formulae A to I above. It may be seen that there is a trend for the yield point to reduce with increasing charge density and with increasing molecular weight for the PolyQuaternium compounds. Table 5 shows the charge density, at two different pH values of 5.3 and 7.01, and the molecular weight for various commercially available PolyQuaternium compounds.

Figure 4 shows the relationship between the yield point, measured in Pascals as for the results of Figure 1, and the amount, in wt% (as supplied) based on the weight of the composition, of a commercially available PolyQuaternium compound, PolyQuat 7. It may be seen that at a concentration of the quaternary polymer of at least about 0.8 wt% for this example, the yield point increases sharply from a low, almost negligible value to a significant value of about 4 to 5 Pascals at a concentration of the quaternary polymer of about 1 to 2 wt% based on the weight of the composition. This demonstrates clearly that the addition of the quaternary polymer to a composition comprising the colloidal dispersion of the clay and the crosslinked polycarboxylate thickener significantly increases the yield point. This achievement of a high yield point provides the many technical and commercial advantages described herein.

**Table 5**

| Charge Density and Molecular Weight of Various PolyQuats | | | |
|---|---|---|---|
| Polymer | meq/gm, pH 5.3 | meq/gm, pH 7.01 | Molecular Weight |
| Merquat™ 295 | 5.78 | 5.72 | 190000 |
| Merquat™ 280 | 2.9 | 2.45 | 450000 |
| Merquat™ 2001 | 0.8 | 0.1 | 1200000 |
| Merquat™ 3330 | 0.1 | -0.5 | 1500000 |
| Merquat™ 100 | 6.19 | 6.19 | 150000 |
| Merquat™ 550 | 3.05 | 3.05 | 1600000 |
| Merquat™ 5 | 0.35 | 0.35 | 4000000 |

### EXAMPLE IV

An additional composition, shown in Table 6, according to the present invention was formed as follows:

**Table 6**

| Ingredients | (wt%) |
|---|---|
| Deionized Water | 52.695 |
| Clay | 0.250 |
| Crosslinked Polycarboxylate Associative Thickener | 4.250 |
| SLES(SO3Na Pareth 145-2EO Sulfate Base-25.5% AI) | 26.002 |
| Cocoamidopropyl Betaine (Coco Fatty Acid, Topped, Hydrogenated) | 8.878 |
| PolyQuat-7 | 1.200 |
| Tetrasodium EDTA-39% AI | 0.200 |
| DMDM Hydantoin | 0.500 |
| Sunflower Oil | 3.000 |
| Petrolatum | 1.420 |
| Silicone Elastomer | 0.080 |
| 25% NaOH (50% in H₂O) | 0.450 |
| 50% Citric Acid | 0.075 |
| Fragrance | 1.000 |

### EXAMPLE V

An additional composition, comprising a shower gel, as shown in Table 7, according to the present invention was formed as follows:

**Table 7**

| Ingredients | Wt % |
|---|---|
| Deionized Water | 62.58 |
| Glycerin | 2.50 |
| Laponite™ XLG | 0.30 |
| PEG 400 | 0.90 |
| NaCl | 1.00 |
| SLES (70%) | 9.37 |
| Aculyn™ 88 | 425 |
| Tetrasodium EDTA (62%) | 0.10 |
| DMDM hydantoin | 0.50 |
| NaOH (50%) | 0.70 |
| Cocoamidopropyl Betaine (30%) | 8.65 |
| PolyQuat 7 | 1.20 |
| Sunflower Oil | 1.50 |
| Petrolatum | 5.00 |
| Maleated Caster Oil (Ceraphyl™ RMT) | 0.10 |
| Fragrance | 0.90 |
| PPG-10 Methyl Glucose Ether | 0.35 |
| Pigment | 0.10 |

In this example, the total emollient concentration of the shower gel was about 6.6 wt%, based on the weight of the composition, the primary emollient being petrolatum (5 wt%) but also including a small amount (0.1 wt%) of esterified oil, in particular maleated oil, most particularly maleated caster oil. In product testing, this composition provided a high degree of skin moisturization as perceived by the user, and did not leave the user's skin feeling tight and dry. The addition of the small amount of the maleated oil enhanced this absense of the user's skin feeling tight and dry as compared to s similar composition containing the same other emollients (petrolatum and sunflower oil) in the same amounts.

Moreover, the composition showed good deposition of both petrolatum and sunflower oil onto a wool swatch, using a conventional testing technique for determining the deposition efficiency of emollients from personal care compositions.

Furthermore, the shower gel of this example also included a viscosity control component, PPG-10 methyl glucose ether, which lowered the viscosity of the composition to a value of about 30,000 to about 40,000 mPas (cps) as compared to the same composition without this additive (which had a viscosity of about 43,0000 mPas (cps)). This composition provides the advantage of being able to provide a high moisturizing benefit to the user, but in a low viscosity composition that is easy to dispense from the shower gel container by the user, and also easy to manufacture, is easy to wash off from the skin and does not cause excessive deposition of the emollient on sanitary ware.

In this example, the structured gel was formed using a specific sequence of steps. In the previous examples, the clay (e.g. Laponite™) was initially dispersed in water to form a colloidal dispersion, then the crosslinked polycarboxylate thickener (e.g. Aculyn™) was added to the colloidal dispersion, and thereafter surfactants, comprising the anionic surfactant (SLES) and the amphoteric surfactant (the betaine surfactant) were then added.

However, in the present example, the clay (e.g. Laponite™) was initially dispersed in water, then the anionic surfactant (SLES) was added prior to the addition of the crosslinked polycarboxylate thickener (e.g. Aculyn™).

Such an order of component addition provides improved formation of the structured gel with increased viscosity and yield point of the final composition.

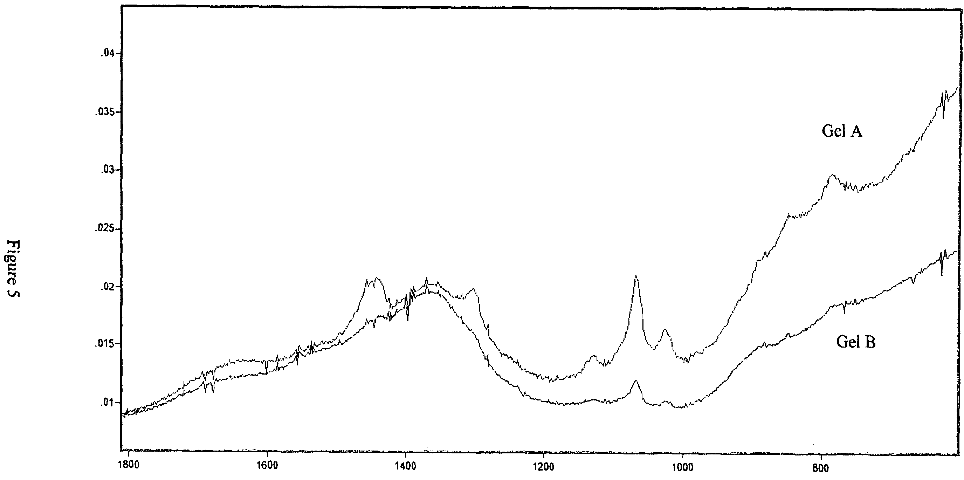

Referring to Figure 5, this shows the relationship between order of addition and chemical interactions of the clay, surfactant, and polycarboxylate. Figure 5 is a plot of intensity vs. wave number (cm⁻¹) as measured using Raman spectroscopy for two structured gels formed in accordance with the present invention. Each structured gel comprised a clay, a crosslinked polycarboxylate thickener and a quaternary polymer, and also an anionic surfactant and an amphoteric surfactant. The Raman spectra were collected with a Renishaw Confocal Raman Instrument with 785 nm laser source. The samples were placed in a sample cup and covered with a glass coverslip. The coverslip helped to prevent the product from spreading out during the measurement. 3 replicate measurements were made with an exposure/integration time of 20 seconds and the replicates were averaged.

Gel A (corresponding to the previous examples) was formulated by initially adding a crosslinked polycarboxylate thickener to an aqueous colloidal dispersion of a clay, with subsequent addition of an anionic surfactant, and then an amphoteric surfactant.

Gel B (corresponding to the present example) was formulated by initially adding an anionic surfactant to an aqueous colloidal dispersion of a clay, with subsequent addition of a crosslinked polycarboxylate thickener, and then an amphoteric surfactant. It may be seen from Figure 5 that the plot of gel B exhibits reduced molecular vibrations as compared to gel A. This indicates that by adding the anionic surfactant to an aqueous colloidal dispersion of a clay, the anionic surfactant and the clay bind together before the addition of the crosslinked polycarboxylate thickener, which then provides more efficient structural support for the bound clay/anionic surfactant molecules and suppresses molecular vibrations.

Accordingly, the order of component addition in accordance with this example can provide an increase in the strength of the network of clay particles, as a result of the interaction with the anionic surfactant, which results in a more structured gel after addition of the crosslinked polycarboxylate thickener. In each case, the quaternary polymer is added after addition of the amphoteric surfactant to the composition, and the benefit agent, in particular the al least one emollient, is added after the structured gel network, including the quaternary polymer, has been formed.

### Example VI

In this Example, the tendency of the composition of the present invention to have a low residue deposition onto a glass surface, representing a surface of sanitary ware, was investigated, and compared to a currently commercially available moisturizing bodywash which exhibited a significantly higher residue deposition.

In particular, glass slides were treated in accordance with the following method with various body cleansing products to be tested:

### Method for Residue Deposition on Glass for Liquid Body Cleansing Products.

1. Scribe glass slides to uniquely identify them for the test. Slide dimensions should be approximately 7.56 cm x 2.49 cm x 0.1 cm and weigh approximately 4.5 g.
2. Rinsed pre-washed slides in the following at room temperature:
   a. Tap water
   b. De-ionized water (less than about 1 µSem)
   c. 200 proof ethanol
   d. Reagent grade Acetone.
3. Weigh out 85 g of water of the desired hardness into a 150 ml Pyrex beaker. (In this case either 100 or 200 ppm, although other levels of water hardness could be used).
4. Add a Teflon coated stir bar.
5. Heat the solution in the beaker to 37°C with the stir bar set to 350 rpm with a hot plate that can be controlled with an attached thermocouple.
6. Remove from the hot plate and add 15 g of product to be tested.
7. Return to the hot plate and continue to maintain temperature at 37°C.
8. Stir with the stir bar at 450 rpm for 6 minutes.
9. Stop stirring and turn off the hot plate, but keep the beaker on the hot plate. During the rest of the procedure the temperature should drop no lower than about 33°C.
10. Place 4 pre-weighed glass slides at an arbitrary angle (generally about 10 to about 30°) in the solution so as not to be touching each other, and not to be directly parallel with the sides of the beaker. This is to maximize the area of the slides exposed to the liquid. In addition, approximately 2/3 of the slide's length will be submerged using this technique to simulate partial exposure to liquid as would be present in a shower environment.
11. Allow the slides to sit in the solution for ten minutes.
12. Lift the slides out of the solution with tweezers and rotate 180 degrees, followed by placement back in the solution as described above.
13. Allow the slides to sit in the solution for an additional ten minutes.
14. Lift the slides out of the solution with tweezers and allow the slides to drain for approximately 5 seconds to one corner, followed by touching the corner of the slide to the edge of the beaker to remove excess fluid without substantially disturbing the surfaces of the slides.
15. Place the slide into a slide holder with a paper absorbent bottom to minimize disturbance of the slide surfaces and allow to air dry for at least 24 hours.
16. Weigh the slides and compare to the pre-washed weight to determine the amount of residue adhering to the slides.
17. Convert the weight of the residue to mg/cm² by dividing the mg of residue by the total surface area of the slide (approximately 39.7 cm²).
18. Calculate the mean mg/cm² and standard deviation of each product treatment.

The results are summarized in Table 8.

**Table 8**

| Formula | % Petrolatum/emollients | Water Hardness (ppm) | Petrolatum Deposition on Glass mg/cm2 | StDev |
|---|---|---|---|---|
| Invention Composition 1 | 5 | 100 | 0.62 | 0.19 |
| Invention Composition 1 | 5 | 200 | 0.64 | 0.22 |
| Comparative Composition | Estimated 48% | 100 | 2.44 | 0.52 |
| Comparative Composition | Estimated 48% | 200 | 2.10 | 1.28 |

Further compositions according to the invention were tested and the results are shown in Table 9.

**Table 9**

| Formula | % Petrolatum | Water Hardness (ppm) | Petrolatum Deposition on Glass mg/cm2 | StDev |
|---|---|---|---|---|
| Invention Composition 2 | 1.5 | 200 | 0.17 | 0.06 |
| Invention Composition 3 | 5 | 200 | 0.59 | 0.21 |
| Invention Composition 4 | 8 | 200 | 0.87 | 0.24 |

The Invention Compositions 1 to 4 are summarized in Table 10.

**Table 10**

| | Inv. Comp. 1 | Inv. Comp. 2 | Inv. Comp. 3 | Inv. Comp. 4 |
|---|---|---|---|---|
| Ingredients | Wt% | Wt% | Wt% | Wt% |
| Deionized Water | 64.29 | 68.78 | 63.73 | 61.58 |
| Glycerin | 2.50 | - | 2.50 | 2.50 |
| Laponite™ XLG | 0.30 | 0.30 | 0.30 | 0.30 |
| PEG 400 | 0.90 | 0.90 | 0.90 | 0.90 |
| C12-C14 Alcohol EO 2:1 Na sulfate (70%) | 9.37 | 9.37 | 9.37 | 9.37 |
| Aculyn™ 88 | 4.25 | 4.25 | 4.25 | 4.25 |
| Tetrasodium EDTA (62%) | 0.10 | | 0.10 | 0.10 |
| Tetrasodium EDTA (39%) | - | 0.23 | - | |
| DMDM hydantoin | 0.50 | 0.60 | 0.50 | 0.50 |
| NaOH (50%) | 0.28 | 0.32 | 0.70 | 0.35 |
| Cocoamidopropyl Betaine (30%) | 8.65 | 8.65 | 8.65 | 8.65 |
| PolyQuat 7 | 1.20 | 1.20 | 1.20 | 1.20 |
| Sunflower Oil | 1.50 | 3.00 | 1.50 | 1.00 |
| Petrolatum | 5.00 | 1.50 | 5.00 | 8.00 |
| Maleated Caster Oil (Ceraphyl™ RMT) | 0.10 | - | 0.10 | 0.10 |
| Fragrance | 0.95 | 0.90 | 0.90 | 0.90 |
| PPG-10 Methyl Glucose Ether | - | - | 0.20 | 0.20 |
| Pigment | 0.70 | - | 0.10 | 0.10 |
| Fruit extract | 0.05 | - | - | - |

The Comparative Composition was the Oil of Olay Ribbons Body Wash product available in commerce from The Procter & Gamble Company, Cincinnati, Ohio, USA which had the following labeled composition: Water, Petrolatum, Sodium Trideceth Sulfate, Mineral Oil, Sodium Lauroamphoacetate, Sodium Chloride, Cocamide MEA, Fragrance, Prunus Amygdalus Dulcis (Sweet Almond) Oil, Hydrolyzed Silk, Guar Hydroxypropyltrimonium Chloride, Glycerin, Citric Acid, DMDM Hydantoin, Acrylonitrile/Methacrylonitrile/ Methyl Methacrylate Copolymer, Isopentane, Sodium Benzoate, PEG-90M, Disodium EDTA, Sodium Hydroxide, Red 7.

It is believed that that product, which is sold as a moisturizing body wash product, has two separate phases, with one phase being a cleaning phase, containing surfactants and the other phase being a moisturizing phase, containing emollients such as the petrolatum, mineral oil and almond oil. It is believed that the petrolatum and emollients content in the entire composition is about 48 wt %. The moisturizing phase has a rather large proportion of petrolatum to provide the necessary moisturizing effect when the entire composition is used as a body wash.

The compositions of the present invention have varying amounts of petrolatum, together with other emollients such as the sunflower oil and the maleated castor oil, but still provide a high degree of moisturization because of the gel structure uniformly supporting the emollients throughout the entire composition.

The compositions of the present invention can provide a similar moisturizing effect to the Comparative Composition. In consumer testing, the bodywash compositions of the present invention having 5wt% petrolatum (corresponding to Invention Compositions 1 and 3) were found to have a similar performance to provide moisturizing of the skin and leaving the skin surface not feeling tight and dry as the Comparative Composition. Increasing the petrolatum content would tend to increase the moisturizing effect.

However, it may be seen that the compositions of the present invention had significantly lower residue petrolatum deposition on the glass surface than the Comparative Composition, even at a relatively high, 8 wt%, petrolatum content. The petrolatum residue deposition for the compositions of the present invention did not change significantly with a change in water hardness, and for the Comparative Composition the petrolatum residue deposition was high at both water harness values.

This Example demonstrates that the structured compositions of the present invention can provide a much more consumer friendly bodywash or shower gel product, which requires less cleaning of sanitary ware surfaces in a user's bathroom, than for a commercial bodywash providing similar moisturizing properties.

Using compositions 2-4 from Table 10, the deposition on wool was measured using the following procedure:
- 1.: Weigh and record vials with caps- use gloves in handling- 5 vials per sample. Number cap and Vials.
- 2.: Wash wool swatches (Worsted Gabardine, Style 541 obtained from Test Fabrics, 415 Delaware Ave, West Pittston, Pa., approximately 68 cm²)with 250 µl of sample (5 swatches per sample).
- 3.: Wash Method: Wash with tap water at about body temperature for 30s making sure that the whole wool swatch area is washed using gloved hands.
- 4.: Wash using continuous circular motions. Rinse under body temperature tap water for 30 sec, alternating sides to ensure complete rinse.
- 5.: Place wool swatch in a laminar flow hood to dry for at least 12 to 15 hours.
- 6.: Using forceps, place swatches in numbered pre-weighed vials, close vials.
- 7.: Take weight to calculate the area of the swatches.
- 8.: Add 10 ml reagent grade acetone and seal and allow to equilibrate overnight.
- 9.: Take weight of vial with swatch and acetone plus cap.
- 10.: Remove swatches using forceps, take weight of vial/cap with acetone only, and use to calculate a correction factor for the amount of acetone and sample lost when the wet swatch is removed from the vial.
- 11.: Uncap vials and leave overnight to evaporate in a chemical exhaust hood.
- 12.: Analyze for petrolatum by GC/MS.
- 13.: Correct for losses of petrolatum by using the weights measured during the different phases of the experiment.

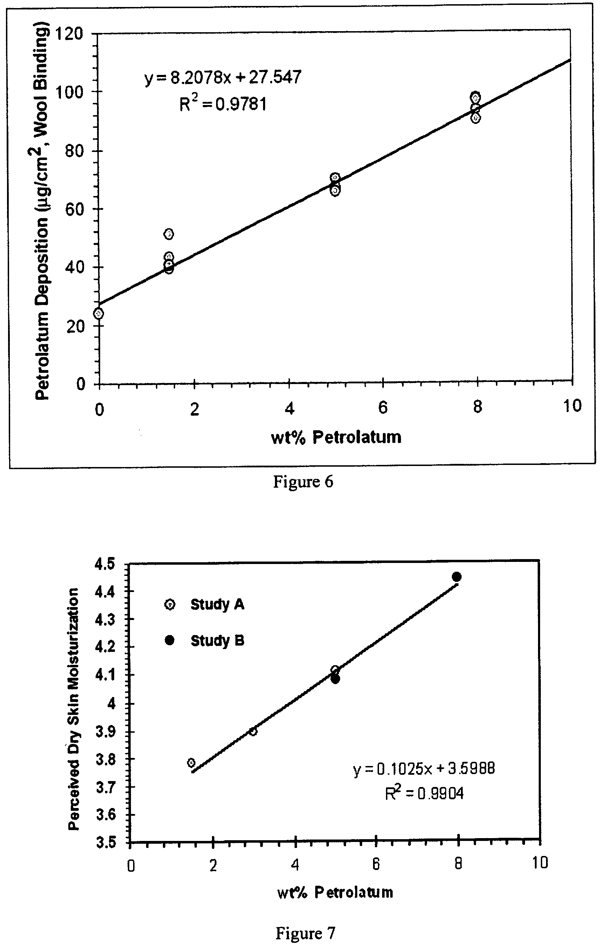

The results are shown in Figure 6. As the level of petrolatum in the composition increases, the level of deposition on wool increases.

Compositions 1-1 to 1-5 shown in Table 11 below were used in a panel study to rate the perceived moisturization to skin after washing and drying. The following procedure was used by the panelists:
1. Wet right arm.
2. Dispense body wash onto left hand.
3. Lather entire right forearm (both sides) for 1 minute.
4. Rinse for 10 seconds.
5. Air Dry.
6. Rate how moisturized the skin feels after drying on a 1 to 6 integer scale (1 not at all moisturized to 6 extremely moisturized).
7. Repeat procedure for left arm using right hand.

**Table 11**

| | Inv. Comp. 1-1 | Inv. Comp. 1-2 | Inv. Comp. 1-3 | Inv. Comp. 1-4 | Inv. Comp. 1-5 |
|---|---|---|---|---|---|
| Ingredients | Wt% | Wt% | Wt% | Wt% | Wt% |
| Deionized Water | 66.13 | 66.13 | 66.03 | 64.13 | 61.13 |
| Laponite XLG | 0.30 | 0.30 | 0.30 | 0.30 | 0.30 |
| Glycerin | 2.50 | 2.50 | 2.50 | 2.50 | 2.50 |
| PEG 400 | 0.90 | 0.90 | 0.90 | 0.90 | 0.90 |
| Sodium Laureth Sulfate (2EO, 70%) | 9.37 | 9.37 | 9.37 | 9.37 | 9.37 |
| Aculyn™ 88 | 4.25 | 4.25 | 4.25 | 4.25 | 4.25 |
| Tetrasodium EDTA (62%) | 0.10 | 0.10 | 0.10 | 0.10 | 0.10 |
| DMDM Hydantoin | 0.50 | 0.50 | 0.50 | 0.50 | 0.50 |
| NaOH (50%) | 0.70 | 0.70 | 0.70 | 0.70 | 0.70 |
| Cocoamidopropyl Betaine (30%) | 8.65 | 8.65 | 8.65 | 8.65 | 8.65 |
| PolyQuat 7 | 1.20 | 1.20 | 1.20 | 1.20 | 1.20 |
| Sunflower Oil with BHT | 3.00 | 1.50 | 1.50 | 1.50 | 1.50 |
| Petrolatum | 1.50 | 3.00 | 3.00 | 5.00 | 8.00 |
| Maleated Castor Oil | 0.00 | 0.00 | 0.10 | 0.00 | 0.00 |
| Fragrance | 0.90 | 0.90 | 0.90 | 0.90 | 0.90 |
| Total | 100.00 | 100.00 | 100.00 | 100.00 | 100.00 |

Two studies were run. The results are shown in Figure 7, which shows the average rating of all panelists at each level of weight % petrolatum (1.5, 3, 5, and 8 weight %). For study A, the average ratings were 3.8 for 1.5 wt% petrolatum, 3.9 for 3 wt.% petrolatum, and 4.1 for 5 wt.% petrolatum. Not shown in Figure 7 is the result for a 3 wt.% petrolatum with 0.1 wt.% Ceraphyl™ RMT, which was 4.8. The results show that as the weight % petrolatum increases, the perceived moisturization increases. Also, the inclusion of Ceraphyl™ RMT increases the perceived moisturization.

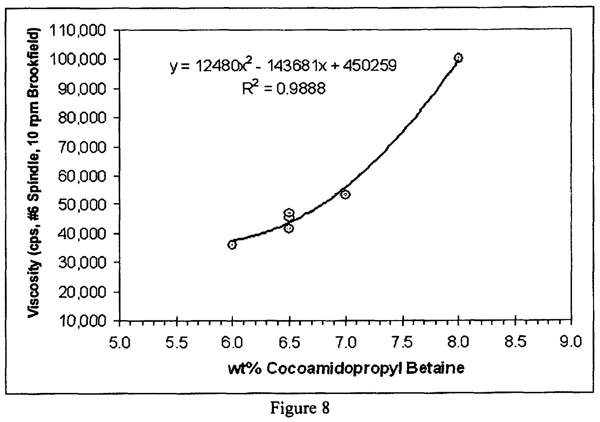

Using Compositions 2-1 to 2-6 shown in Table 12 below, the level of cocamidopropyl betaine was varied to determine the effect on viscosity of the composition. The results are shown in Figure 8.

**Table 12**

| | Inv. Comp. 2-1 | Inv. Comp. 2-2 | Inv. Comp. 2-3 | Inv. Comp. 2-4 | Inv. Comp. 2-5 | Inv. Comp. 2-6 |
|---|---|---|---|---|---|---|
| Ingredients | Wt% | Wt% | Wt% | Wt% | Wt% | Wt% |
| Deionized Water | 51.97 | 51.97 | 51.97 | 51.97 | 50.25 | 48.25 |
| Laponite XLG | 0.30 | 0.30 | 0.30 | 0.30 | 0.30 | 0.30 |
| PEG 400 | 0.90 | 0.90 | 0.90 | 0.90 | 0.90 | 0.90 |
| Sodium Pareth Sulfate (2EO, 25.5%) | 28.78 | 27.78 | 26.78 | 28.28 | 30.00 | 32.00 |
| Aculyn™ 88 | 4.25 | 4.25 | 4.25 | 4.25 | 4.25 | 4.25 |
| Tetrasodium EDTA (62%) | 0.20 | 0.20 | 0.20 | 0.20 | 0.20 | 0.20 |
| DMDM hydantoin | 0.50 | 0.50 | 0.50 | 0.50 | 0.50 | 0.50 |
| NaOH (50%) | 0.50 | 0.50 | 0.50 | 0.50 | 0.50 | 0.50 |
| Cocoamidopropyl Betaine (30%) | 6.00 | 7.00 | 8.00 | 6.50 | 6.50 | 6.50 |
| PolyQuat 7 | 1.20 | 1.20 | 1.20 | 1.20 | 1.20 | 1.20 |
| Sunflower Oil with BHT | 3.00 | 3.00 | 3.00 | 3.00 | 3.00 | 3.00 |
| Petrolatum | 1.43 | 1.43 | 1.43 | 1.43 | 1.43 | 1.43 |
| Silicon Elastomer | 0.07 | 0.07 | 0.07 | 0.07 | 0.07 | 0.07 |
| Fragrance | 0.90 | 0.90 | 0.90 | 0.90 | 0.90 | 0.90 |
| Total | 100.00 | 100.00 | 100.00 | 100.00 | 100.00 | 100.00 |

Additional Inventive Compositions 3-1 to 3-3 that contain tocopheryl acetate are summarized in Table 12 below.

**Table 13**

| | Inv. Comp. 3-1 | Inv. Comp. 3-2 | Inv. Comp. 3-3 |
|---|---|---|---|
| Ingredients | Wt% | Wt% | Wt% |
| Deionized Water | 64.34 | 64.31 | 64.26 |
| Glycerin | 2.50 | 2.50 | 2.50 |
| Laponite™ XLG | 0.30 | 0.30 | 0.30 |
| PEG 400 | 0.90 | 0.90 | 0.90 |
| C12-C14 Alcohol EO 2:1 Na sulfate (70%) | 9.37 | 9.37 | 9.37 |
| Aculyn™™ 88 | 4.25 | 4.25 | 4.25 |
| Tetrasodium EDTA (62%) | 0.10 | 0.10 | 0.10 |
| DMDM hydantoin | 0.50 | 0.50 | 0.50 |
| NaOH (50%) | 0.28 | 0.28 | 0.28 |
| Cocoamidopropyl Betaine (30%) | 8.65 | 8.65 | 8.65 |
| PolyQuat 7 | 1.20 | 1.20 | 1.20 |
| Sunflower Oil | 1.50 | 1.50 | 1.50 |
| Petrolatum | 5.00 | 5.00 | 5.00 |
| Maleated Caster Oil (Ceraphyl™ RMT) | 0.10 | 0.10 | 0.10 |
| Fragrance | 0.95 | 0.95 | 0.95 |
| Tocopheryl acetate | 0.02 | 0.05 | 0.1 |
| Pomegranate extract | 0.05 | 0.05 | 0.05 |
| Total | 100 | 100 | 100 |

It will be appreciated by those skilled in the art that changes could be made to the embodiments described above without departing from the broad inventive concept thereof. It is understood, therefore, that this invention is not limited to the particular embodiments disclosed, but it is intended to cover modifications within the scope of the present invention as defined by the appended claims. For example, the present invention can be used in a variety of portable forms for oral and personal care compositions.

The invention can be further described by the following embodiments:

Embodiment 1 -- A composition comprising:
a. a clay;
b. a crosslinked polycarboxylate thickener;
c. a quaternized polymer;
d. a polyhydric alcohol having a molecular weight of less than about 600; and
e. a surfactant mixture comprising at least one anionic surfactant and at least one amphoteric surfactant;
wherein the composition has a viscosity at room temperature of about 10,000 to about 100,000 mPas (cps), and a yield point at room temperature of greater than about 5 Pa. In various embodiments, the polyhydric alcohol may have a molecular weight of less than about 550 or less than about 500.

The composition of embodiment 1, having a viscosity at room temperature of about 25,000 to about 70,000 mPas (cps), about 30,000 to about 60,000 mPas (cps), about 32,000 to about 48,000 mPas (cps), or about 35,000 to about 45,000 mPas (cps).

The composition of embodiment 1, wherein the synthetic or natural clay is a smectite, for example, chosen from sodium magnesium silicate, lithium magnesium silicate, magnesium aluminum silicate, bentonite or hectorite.

The composition of embodiment 1, wherein the crosslinked polycarboxylate thickener is an anionic hydrophobically modified polymer emulsion.

The composition of embodiment 1, wherein the quaternized polymer has a molecular weight of less than about 2,000,000 and a charge density of less than about 6 meq/g at a pH of about 7. The quaternized polymer may have, for example, a molecular weight of less than about 1,700,000 and a charge density of less than about 4 meq/g at a pH of about 7.

The composition of embodiment 1 may further comprise an emollient chosen from an oil, petrolatum and petrolatum derivative, an elastomer or combination thereof, about 10 to about 70% water, an ingredient chosen from a dye, pigment, emollient, opacifying agent, skin smoothing agent, occluding agent or combination thereof.

The composition of embodiment 1 may further comprise, in addition to the clay structured system, a system chosen from: a spherulite structured system, an acrylate structured system, a water-in-oil emulsion, an oil-in-water emulsion or a polymer.

Embodiment 2 -- personal care composition comprising:
a. a first visually distinct area comprising a synthetic or natural clay; and
b. a second visually distinct area;
wherein at least one of the first or second visually distinct areas comprises a material chosen from a crosslinked polycarboxylate thickener, a quaternized polymer and an antibacterial agent. The visually distinct areas may differ in, for example, color (including the presence or absence of colorant in the respective phases), shade of color, viscosity, opacity or presence or lack of particles and/or inclusions.

The composition of embodiment 2, wherein at least one of the first or second visually distinct area comprises a system chosen from: a spherulite structured system, an acrylate structured system, a water-in-oil emulsion, an oil-in-water emulsion or a polymer. The first visually distinct area may comprise a synthetic or natural clay, a quaternized polymer and a crosslinked polycarboxylate thickener; and the second visually distinct area may comprise a system chosen from: a spherulite structured system, an acrylate structured system, a water-in-oil emulsion, an oil-in-water emulsion or a polymer. The natural clay may be a smectite. The composition may comprise two or more colors, shades of a color, or opacities, and may be in the form of a body wash.

Embodiment 3 -- A personal care composition comprising:
a. about 0.01 to about 5% of a clay;
b. about 0.1 to about 10% of a crosslinked polycarboxylate thickener;
c. about 0.01 to about 5% of quaternary polymer;
d. about 0.1 to about 20% of an emollient;
e. about 0.05 to about 10% of a polyhydric alcohol having a molecular weight of less than about 600; and
f. optionally, about 0.05 to about 5% of an antibacterial agent;
wherein the composition has a viscosity at room temperature of about 10,000 to about 100,000 mPas (cps), and a yield point at room temperature of greater than about 5 Pa. The composition may further comprise about 1 to about 12% of a surfactant. In various embodiments, the polyhydric alcohol may have a molecular weight of less than about 550 or less than about 500. At least one of the first or second visually distinct area may comprise one or more inclusions chosen from a bubble, a bead, a flake, a film, a seed or a particle. The particle may comprise a material chosen from a polymer, a polymeric matrix, glass, plant material, animal material, fiber, glitter, mineral or a combination thereof.

Embodiment 4 -- A method of cleaning a keratinous surface, said method comprising the steps of applying a composition according to the present invention to the keratinous surface and removing said composition from contact with the keratinous surface.

The keratinous surface is chosen from skin, hair or nails. Also contemplated is a method of conditioning a keratinous surface, comprising the steps of:
(I) applying to the keratinous surface a composition comprising:
   a. about 0.01 to about 5% of a synthetic or natural clay;
   b. a crosslinked polycarboxylate thickener;
   c. a quaternary polymer;
   d. a surfactant mixture comprising at least one anionic surfactant and at least one amphoteric surfactant, and
   e. an emollient;
   wherein the composition has a viscosity at room temperature of about 10,000 to about 100,000 mPas (cps), and a yield point at room temperature of greater than about 5 Pa; and
(II) rinsing the composition from the keratinous surface.

Embodiment 5 -- A method of preventing dry skin or dry scalp, comprising the steps of:
(a) applying to the skin or scalp a composition comprising any of the described inventive compositions; and
(b) rinsing the composition from the skin or scalp.

Embodiment 6 -- A method of prolonging delivery of moisture to a keratinous surface, comprising applying to the keratinous surface a composition in accordance with any of the preceding embodiments.

Also contemplated are methods for making any of the structured systems and personal care compositions of the present invention, as well as methods of optimizing the viscosity of a composition to improve filling of systems having varying visibly distinct areas, said method comprising the steps of optimizing the ratio of isopropyl palmitate to cocoamidopropyl betaine.

## Claims

1. A personal care composition comprising:
a. a structured aqueous gel component formed from 0.1 to 1% optionally 0.1 to 0.5% of a clay; 1 to 7.5% of a crosslinked polycarboxylate thickener; and 0.1 to 2% of quaternary polymer, each wt% being based on the weight of the composition; and
b. 0.1 to 25%, based on the weight of the composition, of an emollient component, the emollient component being dispersed in the gel component,
wherein the clay comprises a synthetic layered silicate, optionally the personal care composition is a body wash, a shower gel or a liquid hand soap.

2. A personal care composition according to claim 1, wherein the structured aqueous gel component is comprised in a first visually distinct area, and the composition further comprises:
c. a second visually distinct area comprising a second structured aqueous gel component formed from 0.1 to 1%, optionally 0.1 to 0.5% of a clay; 1 to 7.5% of a crosslinked polycarboxylate thickener; and 0.1 to 2%, optionally 0.1 to 0.2% of quaternary polymer, each wt% being based on the weight of the second visually distinct area, wherein the clay comprises a synthetic layered silicate;
and wherein the emollient component comprises 0.1 to 20% of the weight of the composition, the emollient component being dispersed in at least one of the first and second gel components.

3. A personal care composition according to claim 2 wherein the emollient component is dispersed in both of the first and second gel components.

4. A personal care composition according to any one of claims 2 to 3 wherein each of the first and second structured aqueous gel components has the same composition of clay, crosslinked polycarboxylate thickener and quaternary polymer, and the same amount of the emollient component, and the first and second structured aqueous gel components are visually distinct by comprising different colorant compositions therein.

5. A personal care composition according to any one of claims 2 to 4 wherein the quaternary polymer has a molecular weight of less than 2,000,000 and a charge density of less than 6 meq/g at a pH of about 7, optionally less than 1,700,000 and a charge density of less than 4 meq/g at a pH of about 7.

6. A composition according to any one of claims 2 to 5 wherein at least one of the first or second visually distinct areas comprises one or more inclusions chosen from a bead, a flake, a seed or a particle, wherein the particle comprises a material chosen from a polymer, plant material, glitter, mineral or a combination thereof.

7. A composition according to claim 1 the composition further comprising:
a polyhydric alcohol having a molecular weight of less than 600, optionally less than 550 or less than 500; and
a surfactant mixture comprising at least one anionic surfactant and at least one amphoteric surfactant;
wherein the composition has a viscosity at room temperature of 10,000 to 100,000 mPas (cps), optionally 30,000 to 60,000 mPas and a yield point at room temperature of greater than 5 Pa.

8. A composition according to any preceding claim further comprising an emollient chosen from an oil, petrolatum and petrolatum derivative, an optional elastomer or combination thereof, 10 to 70% water, an ingredient chosen from a dye, pigment, emollient, opacifying agent, skin smoothing agent, occluding agent or combination thereof.

9. A personal care composition according to claim 1, wherein the composition comprises:
0.1 to 20% of the emollient;
0.05 to 10% of a polyhydric alcohol having a molecular weight of less than 600; and
optionally, 0.05 to 5% of an antibacterial agent;
wherein the composition has a viscosity at room temperature of 10,000 to 100,000 mPas (cps), and a yield point at room temperature of greater than 5 Pa.

10. A composition according to claim 9 wherein the composition further comprises 1 to 12% of a surfactant.

11. A method of cleaning a keratinous surface, said method comprising the steps of applying a composition according to any one of claims 1 to 10 to the keratinous surface and removing said composition from contact with the keratinous surface.

12. A method of conditioning a keratinous surface, comprising the steps of:
I) applying to the keratinous surface a composition according to any one of claims 1 to 10 to the keratinous surface; and
II) rinsing the composition from the keratinous surface.

13. A method of preventing dry skin or dry scalp, comprising the steps of:
a. applying to the skin or scalp a composition comprising a composition according to any one of claims 1 to 10; and
b. rinsing the composition from the skin or scalp.

14. A method of prolonging delivery of moisture to a keratinous surface, comprising applying to the keratinous surface a composition in accordance with any one of claims 1 to 10.

15. A method of producing personal care composition comprising:
a. dispersing a clay in water to form a composition;
b. adding an anionic surfactant to the composition in which the anionic surfactant is bound to the clay;
c. adding a crosslinked polycarboxylate thickener to the structured clay component to form a structured phase;
d. adding a quaternary polymer to the structured phase to form a structured gel component; and
e. adding at least one emollient to the structured gel component.

16. A method according to claim 15 wherein at least one amphoteric surfactant is added to the structured phase formed in step (c) before addition of the quaternary polymer in step (d).

17. A method according to claim 16 wherein at least one preservative is added to the structured phase formed in step (c) before addition of the quaternary polymer in step (d) or after the addition of the emollients.

18. A method according to claim 17 wherein the at least one preservative is added to the structured phase formed in step (c) before the addition of the at least one amphoteric surfactant.

## Patentansprüche

1. Körperpflegezusammensetzung, die Folgendes umfasst:
a. eine strukturierte wässrige Gelkomponente, die von 0,1 bis 1 %, gegebenenfalls 0,1 bis 0,5 % einer Tonerde; 1 bis 7,5 % eines vernetzten Polycarboxylat-Verdickungsmittels und 0,1 bis 2 % eines quaternären Polymers gebildet wird, wobei jede Gewichtsprozentangabe auf das Gewicht der Zusammensetzung bezogen ist; und
b. 0,1 bis 25 % einer Emollienskomponente, bezogen auf das Gewicht der Zusammensetzung, wobei die Emollienskomponente in der Gelkomponente dispergiert ist,
wobei die Tonerde ein synthetisches Schichtsilikat umfasst, wobei gegebenenfalls die Körperpflegezusammensetzung eine Körperwaschlotion, ein Duschgel oder eine Flüssighandseife ist.

2. Körperpflegezusammensetzung nach Anspruch 1, wobei die strukturierte wässrige Gelkomponente in einem ersten optisch verschiedenen Bereich enthalten ist und die Zusammensetzung weiterhin Folgendes umfasst:
c. einen zweiten optisch verschiedenen Bereich, der eine zweite strukturierte wässrige Gelkomponente umfasst, die von 0,1 bis 1 %, gegebenenfalls 0,1 bis 0,5 % einer Tonerde; 1 bis 7,5 % eines vernetzten Polycarboxylat-Verdickungsmittels und 0,1 bis 2 %, gegebenenfalls 0,1 bis 0,2 % eines quaternären Polymers gebildet wird, wobei jede Gewichtsprozentangabe auf das Gewicht des zweiten optisch verschiedenen Bereichs bezogen ist, wobei die Tonerde ein synthetisches Schichtsilikat umfasst;
und wobei die Emollienskomponente 0,1 bis 20 % des Gewichts der Zusammensetzung umfasst, wobei die Emollienskomponente in mindestens einer der ersten und der zweiten Gelkomponente dispergiert ist.

3. Körperpflegezusammensetzung nach Anspruch 2, wobei die Emollienskomponente in sowohl der ersten als auch der zweiten Gelkomponente dispergiert ist.

4. Körperpflegezusammensetzung nach einem der Ansprüche 2 bis 3, wobei jede der ersten und der zweiten strukturierten wässrigen Gelkomponente die gleiche Zusammensetzung von Tonerde, vernetztem Polycarboxylat-Verdickungsmittel und quaternärem Polymer und die gleiche Menge der Emollienskomponente aufweist und die erste und die zweite strukturierte wässrige Gelkomponente durch Einbinden unterschiedlicher Farbstoffzusammensetzungen darin optisch verschieden sind.

5. Körperpflegezusammensetzung nach einem der Ansprüche 2 bis 4, wobei das quaternäre Polymer ein Molekulargewicht von weniger als 2.000.000 und eine Ladungsdichte von weniger als 6 meq/g bei einem pH-Wert von etwa 7, gegebenenfalls von weniger als 1.700.000 und eine Ladungsdichte von weniger als 4 meq/g bei einem pH-Wert von etwa 7 aufweist.

6. Körperpflegezusammensetzung nach einem der Ansprüche 2 bis 5, wobei mindestens einer des ersten oder des zweiten optisch verschiedenen Bereichs einen oder mehrere Einschlüsse umfasst, die aus einer Perle, einer Flocke, einem Keim oder einem Teilchen ausgewählt sind, wobei das Teilchen ein Material umfasst, das aus einem Polymer, einem pflanzlichen Material, Glitter, einem Mineral oder einer Kombination davon ausgewählt ist.

7. Zusammensetzung nach Anspruch 1, wobei die Zusammensetzung weiterhin Folgendes umfasst:
einen mehrwertigen Alkohol mit einem Molekulargewicht von weniger als 600, gegebenenfalls weniger als 550 oder weniger als 500 und
ein Tensidgemisch, das mindestens ein anionisches Tensid und mindestens ein amphoteres Tensid umfasst;
wobei die Zusammensetzung eine Viskosität bei Raumtemperatur von 10.000 bis 100.000 mPas (Cps), gegebenenfalls 30.000 bis 60.000 mPas und einen Fließpunkt bei Raumtemperatur von mehr als 5 Pa aufweist.

8. Zusammensetzung nach einem vorhergehenden Anspruch, die weiterhin ein Emolliens, das aus einem Öl, Vaseline und einem Vaselinederivat, einem fakultativen Elastomer oder einer Kombination davon ausgewählt ist, 10 bis 70 % Wasser und einen Inhaltsstoff umfasst, der aus einem Farbstoff, einem Pigment, einem Emolliens, einem Trübungsmittel, einem Hautglättungsmittel, einem Okklusionsmittel oder einer Kombination davon ausgewählt ist.

9. Körperpflegezusammensetzung nach Anspruch 1, wobei die Zusammensetzung Folgendes umfasst:
0,1 bis 20 % des Emolliens;
0,05 bis 10 % eines mehrwertigen Alkohols mit einem Molekulargewicht von weniger als 600 und
gegebenenfalls 0,05 bis 5 % eines antibakteriellen Mittels;
wobei die Zusammensetzung eine Viskosität bei Raumtemperatur von 10.000 bis 100.000 mPas (Cps) und einen Fließpunkt bei Raumtemperatur von mehr als 5 Pa aufweist.

10. Zusammensetzung nach Anspruch 9, wobei die Zusammensetzung weiterhin 1 bis 12 % eines Tensids umfasst.

11. Verfahren zur Reinigung einer Keratinoberfläche, wobei das Verfahren die Schritte des Aufbringens einer Zusammensetzung nach einem der Ansprüche 1 bis 10 auf die Keratinoberfläche und des Lösens der Zusammensetzung aus dem Kontakt mit der Keratinoberfläche umfasst.

12. Verfahren zur Konditionierung einer Keratinoberfläche, das die folgenden Schritte umfasst:
I) Aufbringen auf die Keratinoberfläche einer Zusammensetzung nach einem der Ansprüche 1 bis 10 auf die Keratinoberfläche und
II) Spülen der Zusammensetzung von der Keratinoberfläche.

13. Verfahren zur Verhinderung von trockener Haut oder trockener Kopfhaut, das die folgenden Schritte umfasst:
a. Aufbringen einer Zusammensetzung, die eine Zusammensetzung nach einem der Ansprüche 1 bis 10 umfasst, auf die Haut oder die Kopfhaut und
b. Spülen der Zusammensetzung von der Haut oder der Kopfhaut.

14. Verfahren zur Verlängerung der Abgabe von Feuchtigkeit an eine Keratinoberfläche, das das Aufbringen einer Zusammensetzung nach einem der Ansprüche 1 bis 10 auf die Keratinoberfläche umfasst.

15. Verfahren zur Herstellung einer Körperpflegezusammensetzung, das Folgendes umfasst:
a. Dispergieren einer Tonerde in Wasser, um eine Zusammensetzung zu bilden;
b. Zugeben eines anionischen Tensids zu der Zusammensetzung, in der das anionische Tensid an die Tonerde gebunden wird;
c. Zugeben eines vernetzten Polycarboxylat-Verdickungsmittels zu der strukturierten Tonerdekomponente, um eine strukturierte Phase zu bilden;
d. Zugeben eines quaternären Polymers zu der strukturierten Phase, um eine strukturierte Gelkomponente zu bilden; und
e. Zugeben mindestens eines Emolliens zu der strukturierten Gelkomponente.

16. Verfahren nach Anspruch 15, wobei vor der Zugabe des quaternären Polymers in Schritt (d) mindestens ein amphoteres Tensid zu der in Schritt (c) gebildeten strukturierten Phase zugegeben wird.

17. Verfahren nach Anspruch 16, wobei vor der Zugabe des quaternären Polymers in Schritt (d) oder nach der Zugabe der Emollientien mindestens ein Konservierungsmittel zu der in Schritt (c) gebildeten strukturierten Phase zugegeben wird.

18. Verfahren nach Anspruch 17, wobei das mindestens eine Konservierungsmittel vor der Zugabe des mindestens einen amphoteren Tensids zu der in Schritt (c) gebildeten strukturierten Phase zugegeben wird.

## Revendications

1. Une composition d'hygiène personnelle comprenant :
a. un composant de gel aqueux structuré formé avec entre 0,1 et 1 %, en option entre 0,1 et 0,5 % d'une argile ; entre 1 et 7,5 % d'un épaississant polycarboxylate réticulé ; et entre 0,1 et 2 % de polymère quaternaire, chaque % en poids étant basé sur le poids de la composition ; et
b. entre 0,1 et 25 %, basé sur le poids de la composition, d'un composant émollient, le composant émollient étant dispersé dans le composant de gel,
dans laquelle l'argile comprend un silicate synthétique en couches, en option la composition d'hygiène personnelle est un produit lavant pour le corps, un gel de douche ou un savon liquide pour les mains.

2. Une composition d'hygiène personnelle selon la revendication 1, dans laquelle le composant de gel aqueux structuré est compris dans une première zone visuellement distincte, et la composition comprend outre :
c. une deuxième zone visuellement distincte comprenant un deuxième composant de gel aqueux structuré formé avec entre 0,1 et 1 %, en option entre 0,1 et 0,5 % d'une argile ; entre 1 et 7,5 % d'un épaississant polycarboxylate réticulé ; et entre 0,1 et 2 %, en option entre 0,1 et 0,2 % de polymère quaternaire, chaque % en poids étant basé sur le poids de la deuxième zone visuellement distincte, dans laquelle l'argile comprend un silicate synthétique en couches ;
et dans laquelle le composant émollient comprend entre 0,1 et 20 % du poids de la composition, le composant émollient étant dispersé dans au moins soit le premier soit le deuxième composant du gel.

3. Une composition d'hygiène personnelle selon la revendication 2 dans laquelle le composant émollient est dispersé dans les premier et deuxième composants du gel.

4. Une composition d'hygiène personnelle selon l'une quelconque des revendications 2 à 3 dans laquelle chacun des premier et deuxième composants de gel aqueux structuré a la même composition d'argile, d'épaississant polycarboxylate réticulé et de polymère quaternaire, et la même quantité de composant émollient, et les premier et deuxième composants de gel aqueux structuré sont visuellement distincts en comprenant différentes compositions colorantes dans ceux-ci.

5. Une composition d'hygiène personnelle selon l'une quelconque des revendications 2 à 4 dans laquelle le polymère quaternaire a un poids moléculaire de moins de 2 000 000 et une densité de charge de moins de 6 meq/g à un pH d'environ 7, en option de moins de 1 700 000 et une densité de charge de moins de 4 meq/g à un pH d'environ 7.

6. Une composition selon l'une quelconque des revendications 2 à 5 dans laquelle au moins une des première et deuxième zones visuellement distinctes comprend une ou plusieurs inclusions choisies parmi une bille, un flocon, une semence ou une particule, dans laquelle la particule comprend un matériau choisi parmi un polymère, un matériau végétal, une paillette, un minéral ou une combinaison de ceux-ci.

7. Une composition selon la revendication 1, la composition comprenant en outre :
un alcool polyhydrique ayant un poids moléculaire de moins de 600, en option de moins de 550 ou de moins de 500 ; et
un mélange de tensioactifs comprenant au moins un tensioactif anionique et au moins un tensioactif amphotérique ;
dans laquelle la composition a une viscosité à température ambiante comprise entre 10 000 et 100 000 mPas (cps), en option entre 30 000 et 60 000 mPas et un point d'élasticité à température ambiante supérieur à 5 Pa.

8. Une composition selon l'une quelconque des revendications précédentes comprenant en outre un émollient choisi parmi une huile, un pétrolatum et un dérivé du pétrolatum, un élastomère en option ou une combinaison de ceux-ci, entre 10 et 70 % d'eau, un ingrédient choisi parmi une teinture, un pigment, un émollient, un agent opacifiant, un agent de lissage de la peau, un agent d'occlusion ou une combinaison de ceux-ci.

9. Une composition d'hygiène personnelle selon la revendication 1, dans laquelle la composition comprend :
entre 0,1 et 20 % de l'émollient ;
entre 0,05 et 10 % d'un alcool polyhydrique ayant un poids moléculaire de moins de 600 ; et
en option, entre 0,05 et 5 % d'un agent antibactérien ;
dans laquelle la composition a une viscosité à température ambiante comprise entre 10 000 et 100 000 mPas (cps) et une limite d'élasticité à température ambiante de plus de 5 Pa.

10. Une composition selon la revendication 9 dans laquelle la composition comprend outre entre 1 et 12 % d'un tensioactif.

11. Un procédé de nettoyage d'une surface kératinisée, ledit procédé comprenant les étapes consistant à appliquer une composition selon l'une quelconque des revendications 1 à 10 sur la surface kératinisée et à retirer ladite composition qui est en contact avec la surface kératinisée.

12. Un procédé de conditionnement d'une surface kératinisée, comprenant les étapes consistant à :
I) appliquer sur la surface kératinisée une composition selon l'une quelconque des revendications 1 à 10 sur la surface kératinisée ; et
II) rincer la composition pour la retirer de la surface kératinisée.

13. Un procédé de prévention d'une peau sèche ou d'un cuir chevelu sec, comprenant les étapes consistant à :
a. appliquer sur la peau ou le cuir chevelu une composition comprenant une composition selon l'une quelconque des revendications 1 à 10 ; et
b. rincer la composition pour la retirer de la peau ou du cuir chevelu.

14. Un procédé de prolongement de la distribution d'humidité à une surface kératinisée, comprenant l'application sur la surface kératinisée d'une composition selon l'une quelconque des revendications 1 à 10.

15. Un procédé de fabrication d'une composition d'hygiène personnelle comprenant :
a. la dispersion d'une argile dans de l'eau pour former une composition ;
b. l'ajout d'un tensioactif anionique à la composition dans lequel le tensioactif anionique est lié à l'argile ;
c. l'ajout d'un épaississant polycarboxylate réticulé au composant d'argile structuré pour former une phase structurée ;
d. l'ajout d'un polymère quaternaire à la phase structurée pour former un composant de gel structuré ; et
e. l'ajout d'au moins un émollient au composant de gel structuré.

16. Un procédé selon la revendication 15 dans lequel au moins un tensioactif amphotérique est ajouté à la phase structurée formée à l'étape (c) avant l'ajout du polymère quaternaire à l'étape (d).

17. Un procédé selon la revendication 16 dans lequel au moins un conservateur est ajouté à la phase structurée formée à l'étape (c) avant l'ajout du polymère quaternaire à l'étape (d) ou après l'ajout des émollients.

18. Un procédé selon la revendication 17 dans laquelle le ou les conservateurs sont ajoutés à la phase structurée formée à l'étape (c) avant l'ajout du ou des tensioactifs amphotériques.
